# EUROPEAN PATENT SPECIFICATION

(11) **EP 3 980 769 B1**
(45) Date of publication and mention of the grant of the patent: **22.01.2025**
(21) Application number: 19729713.8
(22) Date of filing: 05.06.2019
(51) Int. Cl.: G01N 31/22, G01N 21/64, G01N 21/78, C09B 57/00, C09B 67/20

(54) **METHOD AND SENSOR FOR DETECTION OF TRIACETONE TRIPEROXIDE (TATP), DIACETONE DIPEROXIDE (DADP), HEXAMETHYLENE TRIPEROXIDE DIAMINE (HMTD) AND HYDROGEN PEROXIDE**
VERFAHREN UND SENSOR FÜR DEN NACHWEIS VON TRIACETON TRIPEROXID (TATP), DIACETON DIPEROXID (DADP), HEXAMETHYLEN TRIPEROXID DIAMIN (HMTD) UND WASSERSTOFFPEROXID
PROCÉDÉ ET CAPTEUR POUR DÉTECTER LE TRIPEROXYDE DE TRIACETONE (TATP), LE DIPEROXYDE DE DIACETONE (DADP), LA DIAMINE DE TRIPEROXYDE D'HEXAMETHYLENE (HMTD) ET LE PEROXYDE D'HYDROGENE

(43) Date of publication of application: 13.04.2022
(73) Proprietor: Bundesrepublik Deutschland, vertreten durch den Bundesminister für Wirtschaft und Energie, dieser vertreten durch den Präsidenten der, 12205 Berlin (DE)
(72) Inventor: BIYIKAL, Mustafa, 12053 Berlin (DE); RURACK, Knut, 12247 Berlin (DE); HELLER, Benedikt, 12489 Berlin (DE)
(74) Representative: Zimmermann & Partner Patentanwälte mbB
(86) International application number: PCT/EP2019/064693
(87) International publication number: WO 2020/244754

(56) References cited:
- WO-A1-2015/169862
- FR-A1- 2 361 654
- JP-A- S59 126 245

## Description

### FIELD AND BACKGROUND

The present invention generally relates to a method for detecting organic peroxide explosives selectively and sensitively.

Commercially available and literature-known systems for detection of peroxide explosives and hydrogen peroxide use weakly fluorescent dyes on hydrophilic carrier materials in presence of strong bases. Hereby these explosives are decomposed to hydrogen peroxide, which reacts with the weakly fluorescent dye, causing a strong fluorescence increase. Different reaction kinetics allow to differentiate between hydrogen peroxide and peroxide explosives (*cf.* US 2014/0193923 A1; Chem. Commun. 2013, 49, 11779-11781). Further, colorimetric sensor arrays have been used to selectively detect peroxide explosives (J. Am. Chem. Soc. 2010, 132, 15519-15521). Such sensor arrays require a combination of more than two sensor layers.

Other techniques used for detecting explosives have certain drawbacks as well: Ion-mobility spectrometry (IMS) requires a radioactive source and exhibits adverse signal drift behavior. Raman spectrometers are often not battery operated and are susceptible to nonspecific fluorescence. Laser based methods are often not battery driven as well and heavily suffer from matrix effects. Classical analytical methods are labor-intensive, costly, time-consuming and not suitable for on-site use. Gas chromatography requires a carrier gas reservoir whereas chemical sensors are typically very selective and allow minimizing cross-sensitivities. However, insufficient broad-band detection is still a major disadvantage. As to colorimetry, reactions of specific sensor layers with peroxide explosives allow their detection merely at higher concentrations. In view of the above, application areas of established detection methods of peroxide explosives are strongly limited.

Against this background fluorescent sensor materials for peroxide explosives are regarded as market leaders. However, cross-sensitivities for hydrogen peroxide and other non-explosive peroxide substances may cause false alarms and thus impair overall effectiveness and market acceptance of fluorescent sensor materials for peroxide explosives.

### BRIEF SUMMARY

The present invention generally relates to a sensor comprising one or more sulfonic acids in combination with a specific dye, which is selected from triarylboranes in acidic (due to the sulfonic acid) hydrophilic or hygroscopic layers. Depending on their nature, the selected molecular sensor provided in a mixed layer has a high selectivity and sensitivity to peroxidic explosives and hydrogen peroxide as indicated by means of either fluorescence "dim-down" / fluorescence attenuation (first aspect) or "light-up" / fluorescence amplification (second aspect) upon contact of the mixed layer with the analyte. The analyte is typically provided in a fluid stream, comprising air or an inert gas, or even a liquid. A gas stream (e.g. air, artificial air, nitrogen, noble gas or any mixture thereof) carrying the analyte(s) may be heated for keeping the analyte(s) solubilized and in order to prevent their deposition (resublimation). The fluid stream is directed (e.g. channeled) towards the mixed layer and a fluorescence of the mixed layer or at least a section of it is measured. The peroxidic explosives (analytes) are disintegrated due to the catalytic action of the sulfonic acid in the mixed layer, hereinafter first mixed layer. Thus, the sensor arrangement comprises at least a first mixed layer. Reaction products interact according to a first aspect of the invention with at least one molecular sensor, the molecular sensor being selected from a triarylamine, a biphenyl diamine, a benzene diamine, and/or a diaryl benzamide. The sulfonic acid is preferably iodinated. If the sulfonic acid is not iodinated, another organo-iodine compound is added into the mixture comprising the first mixed layer. In order to stabilize the first mixed layer with respect to its thickness and position during measurement, the first mixed layer typically comprises an inert matrix, i.e. a hydrophilic compound that is stable under the (typically acidic) conditions. The mixed layer is positioned on a substrate. The substrate is inert and may be optically transparent in at least one of the excitation and emission wavelength ranges. In order to enhance the sensitivity of the detection, another dye - typically a fluorescent dye - may be placed in the light path (between an optical detector and the first mixed layer, either directly into the first mixed layer - if the (fluorescent) dye is stable under the (typically acidic) conditions. Alternatively, the preferably fluorescent dye may comprise a first separate layer, which overlaps with the first mixed layer. It may be stabilized by the same hydrophilic compound and be placed atop or beneath the mixed layer. It may be stabilized even by a hydrophobic compound if placed between the substrate and the mixed layer or - with respect to the first mixed layer - on the opposite substrate surface in case of a transparent substrate. According to a second aspect of the invention, the molecular sensor is a triarylborane dye. The organoiodine compound is not required, though suitable sulfonic acids are chosen among those of the first aspect. The first mixed layer according to the second aspect is typically combined with another hydrophilic or even hygroscopic layer comprising the same or a different sulfonic acid and the same or a different hydrophilic compound. A molecular sensor is not required in this layer. This other layer is designated hereinafter as second separate layer or as catalytic layer. A dye is not required in this layer. In order to establish the required reaction conditions for all layers involved in the sensor arrangement of both aspects, the sulfonic acids need to have a pH of ≤2. Appropriate conditions may be established by adding to and/or supplementing the sulfonic acid(s) with a salt, particularly a salt of a sulfonic acid. Suitable salts are hygroscopic and keep the acid protons hydrolyzed in contact with (humid) air. Preferred cations of the salt are trialkylammonium cations. Others, described below, are suitable as well.

As to the detection mechanism within the mixed layer(s), depending on the nature of the dye, a specific absorption band appears upon interaction with at least one layer whose presence is - according to the **first aspect** - quantified by its quenching influence on the fluorescence of the sulfonic acid (e.g. 1,5-naphthalenedisulfonic acid) or of another fluorescent dye as quantified according to specific fluorescence intensity by exciting within the wavelength range 325-400 nm, e.g. 340-380 nm, and measuring the fluorescence intensity within the wavelength range at 400-850 nm. According to the **second aspect,** the appearance of a new fluorescence band after interaction of the decay products with the triarylborane dye is quantified according to specific fluorescence intensity by exciting within the wavelength range 300-400 nm, e.g. 330-380 nm and measuring the fluorescence intensity within the wavelength range 400-550 nm, e.g. 400-450 nm. The two approaches can be used either separately or in combination with each other for selectively detecting peroxidic explosives and hydrogen peroxide in the air, in dissolved form, and in wipe samples (i.e. at solid surfaces).

In view of the above, a sensor for selective detection of triacetone triperoxide, diacetone diperoxide, hexamethylene triperoxide diamine and hydrogen peroxide in or from their mixture, a method for fabricating same, and a method for detecting an analyte, selected from triacetone triperoxide, diacetone diperoxide, hexamethylene triperoxide diamine, and hydrogen peroxide is suggested.

According to to a first aspect of the present invention the sensor comprises a first mixed layer which is arranged on a substrate, wherein the first mixed layer comprises the following constituents:
- a molecular probe;
- a sulfonic acid; and
- a hydrophilic compound;
wherein according to the invention the molecular probe is a triarylborane. Herein the term "molecular probe" is considered as being synonymous to the terms "molecular sensor", "molecular indicator" and "molecular reporter".

The first mixed layer is prepared by mixing the constituents molecular probe, sulfonic acid, and hydrophilic compound, optionally in a suitable solvent, e.g. ethanol. If the constituents are solid substances, they can be dissolved, e.g., in a 1:1 mixture (volume by volume) of ethanol and ethyl acetate. The mixture of the constituents and the optional solvent is then used for preparing a free standing film or foil or for coating of at least a portion of a substrate surface with a layer of this mixture. The layer may be dried by evaporating the solvent resulting in a dry sensor. The sensors may be kept under preserving conditions before use (e.g. in the dark, at temperatures below 4°C in a refrigerator, in a defined atmosphere, e.g. inert gas composition, defined humidity etc.). The layers of a sensor may as well remain wet, e.g., by using a macromolecular carrier material (hydrophilic compound) or a solvent having a vapor pressure which is below the vapor pressure of water, e.g. below a half the vapor pressure of water or below a tenth of the vapor pressure of water or even below a hundredth of the vapor pressure of water at room temperature.

According to an embodiment the sulfonic acid is selected from a substance according to structure (1), (2), (3), (4) and (5): 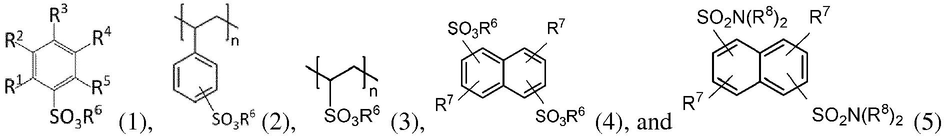 wherein
R1, R², R³, R⁴, and R⁵ are independently from each other = H, Me, Et, alkyl, vinyl, Ph, aryl, I, IO, IOH, IO₂, I(OH)₂, I(OH)OTs, I(OH)SO₃aryl, I(OH)Ph, I(OH)aryl, I(aryl)₂, I(OAc)₂, IO₃, I(OH)₃, CH₂I, Br, Cl, F, NO₂, NH₂, CH₂OH, OH, CF₃, CN, CO₂H, PO₃H₂, OMe, O(alkyl), OPh, O(aryl) or SO₃R⁶;
R⁶ = H, Na, K, Me, Et, alkyl, vinyl, aryl, tetrabutylammonium, tetraoctylammonium, and
n ≥ 8, particularly n= 8-500 or 100-500; preferably n = 150-400;
R⁷ = H, Me, Et, alkyl, vinyl, I, CH₂I, Br, Cl, F, NO₂, NH₂, NHMe, NHEt, NMe₂, NEt₂, NH(alkyl), N(alkyl)₂, NHPh, NPh₂, NH(aryl), CH₂OH, OH, CF₃, CN, CO₂H, OMe, SO₃H; and
R⁸ = H, Me, Et, alkyl, Ph, aryl or any combination thereof.

Herein the word "structure" in connection with a chemical substance is used synonymously to the word "formula". Accordingly, the expression "substance according to structure 1" means "substance according to formula 1", and so forth. Furthermore, the two residues R⁸ at the sulfonamide, e.g. in structure (5), may be identical but may also be different. Correspondingly, the substance according to structure (5) may also be drawn as 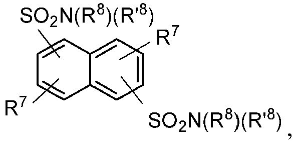 wherein (instead of R⁸ = H, Me, Et, alkyl, Ph or aryl)
R⁸, R'⁸ = H, Me, Et, alkyl, Ph or aryl, independently from each other.

Alternatively, the sulfonic acid is selected from a perfluorosulfonic acid and/or any salt thereof, particularly a sodium or a potassium salt, wherein the perfluorosulfonic acid is selected from a substance according to structure (6) and (7): 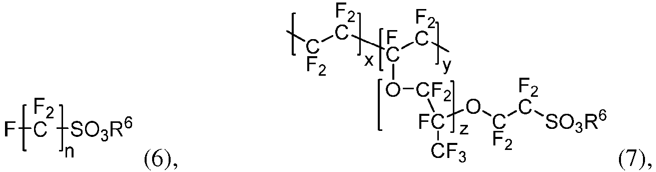 with
- R⁶: = H, Na, K, tetrabutylammonium, tetraoctylammonium, tetraalkylammonium,
- n: ≥ 8,
- x =: y = 50-150, especially 75-125, and
z= 0, 1, 2, 3.

The substance (7) and related materials are also known, e.g., as Nafion^{™}, Hyflon^{™}, Aquivion^{™}, and 3M^{™}-ionomer. Further, the term "iodinated sulfonic acid" and "sulfonic acid" is generally meant to comprise both the free acid and any salt thereof, particularly a sodium salt, a potassium salt or any salt comprising the cations specified further below. Polyvinylsulfonic acid (sodium or potassium salt) according to structure (3) has an average molecular weight of 2-5 kDa, and is available, e.g., at Sigma Aldrich, Catalog Number 278424. Nafion^{®} according to structure (7) as a perfluorinated resin solution is available, e.g., at Sigma Aldrich, Catalog Number 274704. Further examples of suitable sulfonic acids include: octyl sulfonic acid, dodecyl sulfonic acid, octadecyl sulfonic acid, lauryl sulfonic acid, paraffin wax sulfonic acid, naphthalene sulfonic acid, lauryl cyclohexyl sulfonic acid, dodecyl benzene sulfonic acid, polyethylene sulfonic acids of various molecular weights and their salts with a cation selected from: sodium, potassium, ammonium, methylammonium, dimethylammonium, trimethylammonium, tetramethylammonium, tetraethylammonium, tetrapropylammonium, and/or tetrabutylammonium. Furthermore, styrene maleic anhydride co-polymers bearing sulfonate groups on the rings or styrene maleic anhydride co-polymers which have been partially or fully converted to the corresponding imide sulfonates can be used.

Advantageously, the sulfonic acids (and/or their salts) have a very low catalytic effect on the analytes when used in small quantities. However, they show a dramatic increase in the sensitivity of the sensor for triacetone triperoxide. Thus, they contribute to the optimization of the sensor and hence can be considered as constituents of the first mixed layer or any separate layer which will be described further below. If an excess of the sulfonic acids (and/or their salts) is used, starting from 3 equivalents compared to the naphthalenedisulfonic acid and 18 equivalents compared to the molecular probe to 5 and 30 equivalents, the catalytic activity is at its best. If a dye is used and the ratio of the dye to the sulfonic acid is 1/1000 (molar), the catalytic activity of the corresponding layer of the sensor becomes effective. The indicated sulfonic acids serve two purposes: On one hand, the acid has a hydrophilic character, which facilitates the adsorption or absorption of hydrogen peroxide, TATP, DADP and HMTD vapour from air. Secondly, the decomposition of TATP, DADP and HMTD vapours to hydrogen peroxide is catalyzed by the sulfonic acid. Therein, any salt of the sulfonic acid is used in order to keep (turn) the acid in(to) a liquid (protonated) state. Preferably, the salt is used merely in the second separate layer, i.e. in a catalytic layer, downstream of the first mixed layer with respect to a fluid stream which potentially contains an analyte. However, if a salt of the sulfonic acid is used together with a (free) sulfonic acid, said salt can at least partially substitute or even replace the hydrophilic compound.

Furthermore, compounds comprising at least 8 carbon atoms, such as, e.g., perfluorooctanesulfonic acid 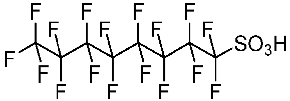 (CAS: 1763-23-1), telomers such as 6:2 1H,1H,2H,2H-tridecafluorooctane-1-sulphonic acid 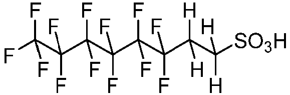 (CAS: 27619-97-2) or 8:2 1H,1H,2H,2H-perfluorodoecansulfonic acid 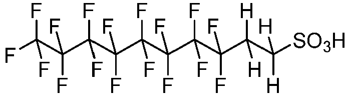 (CAS: 39108-34-4) can be used in the suggested mixed layer or in any other layer (e.g. a catalytic layer such as the second separate layer described further below, as well. The shorter the chain length, the less the vapour pressure of the substance. In view thereof, a chain length of at least 8 is preferred for preparing a stable mixed layer on the substrate.

Advantageously, these compounds are commercially available as they are produced on an industrial scale and are widely applied, e.g. in textile, carpet and leather treatment (water and dirt proofing), as surfactants, fire-fighting foams and for paper grease-proofing treatments. Further, acrylamido(methyl)propylsulfonic acid, as well as the polymer poly(2-acrylamido-2-methyl-1-propanesulfonic acid) may be used for their pronounced hydrophilicity and acidity.

Advantageously, the above indicated sulfonic acids serve two purposes. On one hand, the hydrophilic character of the acid facilitates adsorption or absorption of hydrogen peroxide or TATP, DADP and HMTD vapour from air. Second, the sulfonic acid catalyzes the decomposition of, e.g., TATP to hydrogen peroxide. Among sulfonic acids iodinated arylsulfonic acids are preferred. In general, acids according to formulas (1)-(7) can be employed for the decomposition of TATP. Advantageously, the substance according to structure (4), (5) and biaryls of structure (1) is also fluorescent and its emission band overlaps with the absorption band of the newly formed (colored) species that is the product of the reaction of the organic peroxides with the mixed layer. A decrease in fluorescence of the fluorescent sulfonic acid (e.g. according to any of structures (1)-(7) or the perfluoroalkane sulfonic acids and telomers) can thus be measured. In the reaction, e.g. a iodobenzenesulfonic acid according to structure (1) is oxidized by hydrogen peroxide, forming from the molecular sensors new absorbing (colored) species. Substances according to structures (1)-(7) are compounds with high acidity which can increase the decomposition of TATP, DADP and HMTD, leading to an even higher sensitivity of the sensor layer for the indicated explosives.

Accordingly, following an embodiment, the sulfonic acid is a naphthalenedisulfonic acid according to structure (4) and (5), i.e. one of said acids and/or an ester, an amide or a salt thereof. Therein the cation of the salt is selected from: sodium, potassium, ammonium, methylammonium, dimethylammonium, trimethylammonium, tetramethylammonium, tetraethylammonium, tetrapropylammonium, tetrabutylammonium and/or tetraoctylammonium, wherein
- R⁷: = H, Me, Et, alkyl, vinyl, I, CH₂I, Br, Cl, F, NO₂, NH₂, NHMe, NHEt, NMe₂, NEt₂, NH(alkyl), N(alkyl)₂, NHPh, NPh₂, NH(aryl), CH₂OH, OH, CF₃, CN, CO₂H, OMe, SO₃H; and
- R⁸: = H, Me, Et, alkyl, Ph, aryl or any combination thereof.

Thus, for instance, 1,3-naphthalenedisulfonic acid, 1,4-naphthalenedisulfonic acid, 1,5-naphthalenedisulfonic acid, 1,6-naphthalenedisulfonic acid, 1,7-naphthalenedisulfonic acid, 2,6-naphthalenedisulfonic acid, 2,7-naphthalene-disulfonic acid, 2-amino-1,5-naphthalenedisulfonic acid, 4-amino-1,5-naphthalenedisulfonic acid, 3-amino-7-nitro-1,5-naphthalenedisulfonic acid, or a sodium, potassium, ammonium or tetraalkylammonium salts, ester or amide thereof are suitable sulfonic acids. Of importance is hereby that the used salt, ester or amide show a higher fluorescence quantum yield than the acid. When using salts, their high acidity and the resulting ability of the sensor to hydrolyze the analyte upon contact, are important. Advantageously, the compounds of structure 5 have high quantum yields.

According to an embodiment, the sulfonic acid is selected from a substance according to structure (1) and
- R¹ and/or R⁵ =: H, I, Br, Cl, F, Me, OMe, NO₂ or CN;
- R² and/or R⁴ =: H, I, Br, Cl, F, Me, OMe, NO₂ or CN; and
- R³ =: H, I, Br, F, Cl, Me, NO₂ or CN; independently from each other;
preferably
- R¹ and/or R⁵ =: H or I;
- R² and/or R⁴ =: H or I; and
- R³ =: H or I; independently from each other.

Advantageously, e.g., 4-iodobenzenesulfonic acid is a compound according to structure (1) which is oxidized by reaction with hydrogen peroxide and forms from the molecular sensors the light absorbing species. According to our present understanding, the mechanism for the complete reaction of TATP with the sensor layer is as follows: TATP in gas phase is adsorbed at the sensor layer. Then, the sulfonic acids, especially 4-iodobenzenesulfonic acid, decompose the adsorbed TATP to hydrogen peroxide. Hydrogen peroxide stays absorbed in the layer because of the hygroscopic and hydrophilic properties of the iodobenzenesulfonic acid and the carrier material, i.e. the hydrophilic compound, e.g. a polymer of the HydroMed D Series. Even though the exact mechanism is not fully proven yet, it is strongly suggested that it occurs as follows: Hydrogen peroxide oxidises the iodine of 4-iodobenzenesulfonic acid to a hypervalent iodine compound. This oxidised intermediate then reacts with the sulfonic acid group of another 4-iodobenzenesulfonic acid to form a [hydroxy(sulfonyloxy)iodo]arene similar to Koser's reagent. This hypervalent iodine then oxidises the molecular probe forming a radical cation.

According to an embodiment, the hydrophilic compound comprises a hydrophilic polymer selected from: a block copolymer comprising at least a hydrophilic block; a hydrophilic polyurethane; and an aliphatic polyether-based polyurethane.

Advantageously, these polymers are hydrophilic and provide the appropriate environment in order to allow free protons of e.g. a sulfonic acid which is present in the first (or second) mixed layer as well. Advantageously, the hydrophilic polyurethane carrier materials can absorb water and hydrogen peroxide. This characteristic prolongs the residence time of hydrogen peroxide in or on the sensor layer.

According to an embodiment, the block copolymer comprising at least a hydrophilic block is a nonionic triblock copolymer comprising units of polypropylene oxide) and poly(ethylene oxide).

Such block-copolymers are known for their hydrophilicity and stability in the presence of the sulfonic acids.

According to an embodiment, the hydrophilic polyurethane is selected from commercially available brands. Trade names of suitable hydrophilic polyurethanes, especially of ether-based hydrophilic polyurethanes, are e.g., Hydromed^{™}-D1, -D2, -D3, -D4, -D6, -D640, Hydromed^{™}-D7, and HydroSlip C. Other hydrophilic thermoplastic polyurethane elastomers suitable as a hydrophilic compound, i.e. as inert carrier material are, e.g., HydroThane^{™}, Nanosan^{®}. Trade names of other hydrophilic, especially of aliphatic polyether-based thermoplastic polyurethanes which are suitable as carrier materials are Tecoflex^{™} and Tecophilic^{™}.

Advantageously these hydrophilic polymers are stable under UV-illumination and do not show yellowing by aging or heating. Furthermore, they readily form hydrogels by rapidly absorbing water. These polymers and resulting hydrogels also easily absorb hydrogen peroxide. Surprisingly they withstand possible oxidation by hydrogen peroxide which ensures their suitability as inert carrier materials for the analytes and their reaction products. Polymers of the HydroMed D series have a percental water content of from 30 to 95% and a percental linear expansion from 10 to 180%. Advantageously, they provide a good adsorption and absorption capacity of a corresponding sensor layer for hydrogen peroxide and provide certain porosity of the sensor layer after drying, i.e. an enhanced surface for adsorption of the analytes and their reaction products. For polymers of the HydroMed-D series the following weight ratios have been proven to be suitable: 1.0-2.0 g / 20-25 g / 2-3 g. In particular, weight ratios of 2.0 g of the polymer : 22 g EtOH : 2.5 g H₂O can be used.

Trade names of suitable poloxamers are, e.g., Synperonic^{(R)}, Pluronic^{(R)}, and Kolliphor^{(R)}. In particular, the hydrophilic polymer may be selected, e.g., from poloxamer 188, Pluronic^{(R)} F-68, Pluronic^{(R)} F-108, poloxamer 407, Pluronic^{(R)} F-127, and Pluronic^{(R)} P-123. From experimental results follows that these polymers are inferior in comparison to, e.g., polymers of the Hydromed^{™} series if used as carrier substances in the suggested sensor layer.

Advantageously, the poloxamers have low melting points. Thus at temperatures above 50°C they form liquids which can better uptake hydrogen peroxide than solids.

According to an embodiment other hydrophilic polymers suitable as carrier material are poly(ethylene glycols) having a molecular weight of Mₙ = 35.000, 20.000, 12.000, 10.000, 6.000, 2.500, etc.. Therein, and further below, Mₙ is the total weight of all the polymer molecules in a given sample, divided by the total number of polymer molecules in the sample. Other suitable hydrophilic polymers are poly(ethylene glycol) methyl ether (Mₙ = 35.000, 20.000, 12.000, 10.000, etc.), poly(ethylene glycol) dimethyl ether (Mₙ = 35.000, 20.000, 12.000, 10.000, etc.), poly(ethylene glycol) dialkyl ether (Mₙ = 35.000, 20.000, 12.000, 10.000, etc.), poly(ethylene glycol) diacrylate (Mₙ = 35.000, 20.000, 12.000, 10.000, 6.000 etc.) and co-polymers comprising at least one of the above. Further, poly(ethylene glycol)dimethacrylates (Mₙ = 35.000, 20.000, 12.000, 10.000, 6.000 etc.), polyvinyl alcohol, polyvinyl pyrrolidone, poly(diallyldimethylammonium chloride) and co-polymers comprising at least one of the above. Furthermore, their mixtures with porous materials or a solid foam, wherein the porous material and the solid foam are selected from: foamed silica, a mesoporous silica, and a polyHIPE can be used.

According to this embodiment, polyHIPE comprises porous polymers synthesized within high internal phase emulsions (HIPE), comprising e.g. poly(divinylbenzene) polyhipe polymers, styrene-divinylbenzene polyhipe polymers, dicyclopentadiene polyhipe polymers, or 2-ethylhexyl polyacrylates or their mixtures. The cellular structure of a polyHIPE is isotropic and thus quite different from the oriented structure of commercial blown and extruded foams. PolyHIPE polymers typically have a cellular structure with volume fractions from 0.2 to 0.04, cell diameters from 5 to 25 µm and intercellular pore diameters from 0.5 to 10 µm. Advantageously, the mentioned substances are commercially available and can be used at low costs. Advantageously, mesoporous silica materials or their mixtures provide for an enhanced (contact) surface and thus improve sensitivity for the analytes. For instance, commercially available polyphosphates can be used as carrier materials.

Different hydrophilicity and hygroscopicity can support the adsorption and absorption of TATP, DADP and HMTD and hydrogen peroxide (i.e. the analytes) by the layer comprising the polymer.

According to an embodiment, the sensor comprises a substrate which is selected from: a glass, a metal, a polymer, a mineral, a ceramic, or a composite comprising at least one of them; wherein the polymer comprises a cyclic olefin polymer (COP), a cyclic olefin copolymer (COC), a polyethylene (PE), a polypropylene (PP), a polyvinyl chloride (PVC), a polystyrene (PS), a polytetrafluoroethylene (PTFE), a polymethylmethacrylate (PMMA), a polyacrylonitrile (PAN), a polyamide, an aramide i.e. aromatic polyamide, a polyetherketone (PEK), a polycarbonate (PC), a polyethylene terephthalate (PET), preferably a cyclic olefin polymer, a cyclic olefin copolymer and/or a polycarbonate.

As to the selection of the substrate, it is important that it is not fluorescent or that the fluorescence can be "erased", e.g. by exposure to UV irradiation. If a polymer is used as the substrate, e.g., a cyclic olefin polymer (COP) (see e.g.: Microfluid. Nanofluid. 2010, 9, 145-161), a range of manufacturers offers suitable materials under various brand names (e.g. Apel, Arton, Topas, Zeonex and Zeonor). Some of these (Apel and Topas) are made from more than one kind of monomer and are therefore also designated as cyclic olefin copolymers (COCs), which are fluorescent. However, their fluorescence can be erased upon UV irradiation. The substrate material is selected to be heat resistant at least up to 70 °C. Therefore, for polymers, the glass transition temperature (T_{g}) is of high importance and should be ≥ 70 °C. Advantageously, these polymers can be shaped to comprise fluid-guiding structure, e.g. a channel, either by hot embossing, extrusion, or injection molding.

According to an embodiment, the substrate has a transparency of at least 50% in a wavelength range between 300 nm and at least 800 nm.

According to another embodiment, in the sensor according to any of the previous embodiments, a shape of the substrate encompasses a slide, a plate, a disc, a wall of a channel, a tube, a pipe, a capillary, a porous solid - e.g. a sintered glass frit, a sheet, a slab, a foil, a paper, a grid, a net, a woven or unwoven textile, an open porous sponge, a thread, a filament, a wire, a rod, or a stick.

So far the sensor layer is deposited in notches (indentations, wells) of a "*lab on a chip"* which has round wells with a typical diameter of 1 mm and a depth of 0.2 mm. On the top of the chip there are channels connecting the different positions of the sensor layers. Right before the notches the channels are formed downwards, so the air stream will directly be guided on the notch with the sensor layer and not just only flow over it. Thus, the interaction of the gas stream with the sensor layer is increased, which has positive effects on the sensitivity. According to another design of the lab on a chip, cavities of a diameter of about 1 mm and a depth of about 0.4 mm are interconnected by channels for guiding the analyte carrying gas stream and to form a microfluidic system. Advantageously, with the indicated polymers it is very easy to produce microfluidic systems, e.g., by injection molding, comprising channels etc. in a chip, which may be configured to control the air flow and to have a positive effect on the sensitivity. As known to the skilled person, any of the indicated materials appropriately shaped can be suitable arranged within, e.g., a microfluidic system or in a measurement device, comprising a compact arrangement of analyte sensitive layer(s) - the sensor, a light source and a detector. Typically, the device comprises a channel for allowing a fluidic contact of a fluid stream - which potentially carries one or several analytes - with the sensor, while allowing fluorescence measurement during or after said contact.

According to an embodiment, the suggested sensor further comprises an excitation light and an optical detector, wherein main optical axes of the excitation light and of the optical detector are arranged with respect to each other within an angle of less than 45 °, wherein an angle of the main optical axis of the optical detector with respect to a surface normal of the substrate is ≤ 45 °, wherein the surface normal is a vector perpendicular to the tangent plane of the surface of the substrate at a point of incidence which is defined by a position of the main optical axis of the optical detector at the surface of the substrate.

A preferred configuration of the optical measurement setup includes a measurement in reflection and/or backscatter mode. An angle between the excitation beam path and the fluorescence beam path is preferably between 20 and 60 °, typically between 30 and 50 °. The angle between the beam paths and the surface of the sensor material (any first or second layer on the substrate) is between 60 and 120 ° (in backscatter mode). The instrumental design of a preferred measurement setup can optionally measure in an epi-fluorescence mode or collect signals of backscattering. The incidence angle of the excitation light is about 60 ° with respect to the substrate surface (i.e. 30 ° with respect to the surface normal) and the fluorescence light is collected at a solid angle of ± 30 ° to the surface normal in backscattering. Typical thicknesses of a layer of the sensor are <1 mm, preferably 0.1-400 µm, more preferably 1-100 µm. According to this embodiment, a fluorescence signal emitted by at least one layer of the sensor is measured in epi-illumination mode. However, a fluorescence may also be measured in a trans-illumination mode, even simultaneously with an epi-fluorescence mode, which offers the advantage of, e.g., a compact design for a simultaneous detection of different analytes.

According to an embodiment, the first mixed layer comprises: a iodinated sulfonic acid and/or a mixture of a sulfonic acid with an organoiodine compound, if the sulfonic acid does not comprise any iodine containing substituent.

Advantageously, hydrogen peroxide - if present - first reacts with the sulfonic acid group. According to our present understanding the thus evolving intermediate then reacts, probably intermolecularly, with the iodine of a second molecule of the substance sulfonic acid. This intermediate is then apparently reduced by the molecular probe thus forming the light absorbing species as described above. Therefore, the presence of at least one residue represented by iodine is important. According to typical embodiments, the sulfonic acid is selected from benzenesulfonic acid, a benzenesulfonic acid derivative, a iodobenzenesulfonic acid (e.g. 2-iodobenzenesulfonic acid, 3-iodobenzenesulfonic acid, 4-iodobenzenesulfonic acid, 2,4-diiodobenzenesulfonic acid, 3,5-diiodobenzenesulfonic acid), and a derivative thereof. Especially preferred sulfonic acids are 4-iodobenzenesulfonic acid, 1,5-naphthalenedisulfonic acid, and 4-toluenesulfonic acid. Preferably, 4-iodobenzenesulfonic acid is used in a mixture with 1,5-naphthalenedisulfonic acid and/or 4-toluenesulfonic acid. If the selected sulfonic acid does not contain an iodine substituent, an iodine bearing substance is preferably added into the mixture for forming the analyte sensitive layer. Thus, in such case in total 4 different substances are mixed, preferably with a volatile organic solvent or solvent mixture, for preparing the first mixed layer or the fluorescence sensor: a molecular probe a iodinated sulfonic acid or a sulfonic acid / an organoiodine compound and a hydrogel forming carrier material. Their molar ratio is preferably in the range: 0.1-0.2 / 2.5-10 / 2.5-10 / 0.8-1.2. More preferably their molar ratio is the same as for the three component mixture: molecular probe : organo-iodine-compound : sulfonic acid : naphthalenedisulfonic acid = 0.1-0.2 : 0.4-0.6 : 0.4-0.6 : 0.8-1.0, e.g., 0.16 : 5.51 : 5.51 : 1.00.

Advantageously, a mixture of 4-iodobenzenesulfonic acid and 1,5-naphthalenedisulfonic acid exhibits a higher efficiency of and sensitivity for detection of TATP and hydrogen peroxide. 1,5-Naphthalenedisulfonic acid acts as a dye which shows clearly a decline in fluorescence intensity with TATP and hydrogen peroxide, while the fluorescence intensity increases in the presence of water. That allows for discriminating between hydrogen peroxide and water. The presence of 4-toluenesulfonic acid allows lowering the viscosity of the acid mixture. For instance, the lower viscosity of its mixture with 1,5-naphthalenedisulfonic acid advantageously allows for higher efficiency and sensitivity of the reaction with hydrogen peroxide (other peroxides show a drastically reduced reaction/fluorescence decrease) and TATP.

According to an embodiment, the organoiodine compound is selected from substances according to structure (8) and (9) indicated below: 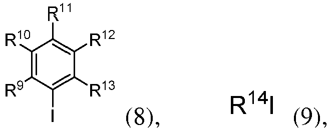 wherein
R⁹, R¹⁰, R¹¹, R¹², and R¹³ are independently from each other = H, Me, Et, alkyl, vinyl, alkynyl, Ph, aryl, I, IO, IOH, IO₂, I(OH)₂, I(OH)OTs, I(OH)SO₃aryl, I(OH)Ph, I(OH)aryl, I(aryl)₂, I(OAc)₂, IO₃, I(OH)₃, CH₂I, Br, Cl, NO₂, NH₂, CH₂OH, OH, CF₃, CN, CO₂H, PO₃H₂, O(alkyl), OPh, O(aryl) or OMe, and R¹⁴ = alkyl, alkenyl, alkynyl;
preferably
   - R¹⁰ and/or R¹² =: H, I;
   - R⁹ and/or R¹³ =: H, I; and
   - R¹¹ =: H, I;
   independently from each other.

If the sulfonic acid selected from substances (1), (2), (3), (4), (5), (6) and/or (7) does not contain any iodine substituent, an organic iodine additive selected from (8) and/or (9) is added into the mixture which is used to generate at least the first mixed layer, and, optionally, also the second mixed layer. Advantageously, the electron donating/withdrawing substituents of the indicated iodo compounds positively affect the oxidation of iodine, thus increasing the sensitivity of the sensor layer. As indicated above, without iodine no fluorescence dim-down could be observed. In view thereof, iodine compounds are favorable present within the analyte sensitive layer(s) in order to enhance the sensitivity of the fluorescence measurement.

According to an embodiment, the sensor further comprises a fluorescent dye, which is either homogenously distributed within the mixed layer (first mixed layer or second mixed layer) or which constitutes a first separate layer on top or beneath the corresponding mixed layer, wherein the fluorescent dye comprises an absorption maximum within a range of 340-550 nm, in particular measuring the fluorescence intensity within the wavelength range at 400-850 nm. Advantages arise with respect to light sources in commercially available fluorescence readers which provide light within the required wavelength ranges.

Said separate layer, hereinafter first separate layer, may comprise an overlapping layer, which is arranged adjacent to the first mixed layer, wherein a fluorescence of the fluorescent dye is reduced by the strongly absorbing species which is formed by a reaction of the analyte with the molecular probe and the organoiodine compound, e.g. 4-iodobenezenesulfonic acid. Advantageously, the attenuation of the fluorescence intensity of the fluorescent dye can be measured in order to detect the concentration of the analyte. Theoretically, one could also measure a transmission and/or absorption signal(s) - and abstain from any fluorescent dye. However, for the very thin layers of the sensor and or for the preferably used epi-fluorescence measurement geometry, this would lead to a significantly inferior sensitivity. Typically, the thickness of the layers of the sensor is below 1 mm, more typically between 0.1 µm and 400 µm, preferably between 1 µm and 100 µm.

All dyes that are stable in the presence of the acid and are excited or emitted in the range of absorption maxima are considered to be suitable dyes. If the dye is outside the layer or in hydrophobic nanoparticles or microparticles like polystyrene inside the acidic mixed layer, the stability against the acid is not important. Naphthalenedisulfonic acids, their salts, esters, amides and amines can be used as the mentioned fluorescent dye (excitation at 340-380 nm). In other words, preferably, a naphthalenedisulfonic acid, their esters, amides, amines and their salts can be used. Moreover, dyes with the structure of rhodamine 6G can be used.

Advantageously, all dyes can be used which can be excited at wavelengths below 400 nm and which fluoresce either in the range 420-520 nm or 600-850 nm. In addition, these dyes can be used in all conceivable formulations, i.e. dissolved, as a film, in a particle, as a solid, etc. Preferably, fluorescent triarylamines and naphthalenedisulfonic acid can be used. Moreover, dyes having the structure of rhodamine 6G can be used and fluorescent sulfonic acid compounds (such as 1,5-naphthalenedisulfonic acid (4), amide (5) or biaryl compounds of structure (1)) can also serve as sources of fluorescence emission. Instead of the above mentioned 1,5-naphthalenedisulfonic acid other fluorescent molecules, whether as dry powder, solution or even encapsulated, e.g., in polystyrene nanoparticles, may be used for excitation, i.e. as emission sources. Also, direct light of a suitable LED, or the backscattering of any other excitation light in the sensor layer, e.g. by a reflection from the backside of the layer, may serve as light source to generate emission. In particular, such dyes are preferred whose excitation range is at shorter wavelength than the absorption band of the sensor layer used and whose emission range overlaps with the absorption band of the sensor layer. Alternatively, dyes can be used whose excitation and emission range both overlap with the absorption band of the sensor layer or dyes whose excitation range overlaps with the absorption band of the sensor layer and whose emission range lies at a higher wavelength. This absorption band emerges when peroxides interact with the relatively colorless sensor layer. From a practical point of view, dyes which can be excited at around 365 nm are preferred, as the fluorescence reader is equipped with a corresponding light source (e.g. a LED). Advantageously, one reader could be used for all sensor layers, remarkably also for layers which are sensitive for TNT and related explosives. To summarize, a suitable fluorescent dye or any other light emitting compound (excited in a range of 350-370 nm or at 365 nm) can be used and be placed in the sensor layer, on top of it or on the opposite site of the (translucent or transparent) substrate. The only importance hereby is that the emission spectrum of the fluorescent compound shall overlap the absorption spectrum of the light absorbing species as much as possible.

Thus, according to a preferred embodiment, in the sensor the fluorescent dye is selected from: a naphthalenedisulfonic acid, and/or a salt thereof, and/or an ester of the naphthalenedisulfonic acid, and/or an amide of the naphthalenedisulfonic acid.

According to the present invention the molecular probe is selected from a triarylborane, the sensor further comprising a second separate layer adjacent to the first mixed layer, the second separate layer comprising:
- a sulfonic acid, or
- a sulfonic acid and a salt of the sulfonic acid, or
- a sulfonic acid and the hydrophilic compound, or
- a sulfonic acid, a salt of the sulfonic acid and the hydrophilic compound;
wherein a cation of the salt of the sulfonic acid is selected from: a sodium, a potassium, an ammonium, a methylammonium, a dimethylammonium, a trimethylammonium, a tetramethylammonium, a tetraethylammonium, a tetrapropylammonium, a tetrabutylammonium and/or tetraoctylammonium cation; and wherein the second separate layer does not overlap with the first mixed layer.

Typically, hygroscopic and water-absorbing materials serve as carrier materials for the selected acid(s) and the triarylborane dyes. Most of the sulfonic acids identified above are crystalline solids. As air humidity increases, they absorb water from the air and pass through the excess water of crystallization into the liquid phase. The catalytic activity of the acid depends on whether it is in liquid or solid form. There are no free protons in the solid form. Therefore, the catalytic activity of the acid in solid form is low. In dissolved form, however, free protons are present, which means that the acid has a high catalytic activity. To ensure that the catalytic activity of the acid is not dependent on environmental conditions or that the acid does not crystallize, hydrogel/acid mixtures or a mixture of acid and the corresponding tetrabutylammonium salt (TBA salt) of the acid are used. A mixture of hydrogel/TBA sulfonate/sulfonic acid and porous silica/TBA sulfonate/sulfonic acid can be used as well. Hydrogels are known for their ability to absorb water. The TBA salts of the acids are hygroscopic and attract humidity from the environment and thus keep the catalyst layer in liquid form even at higher temperatures.

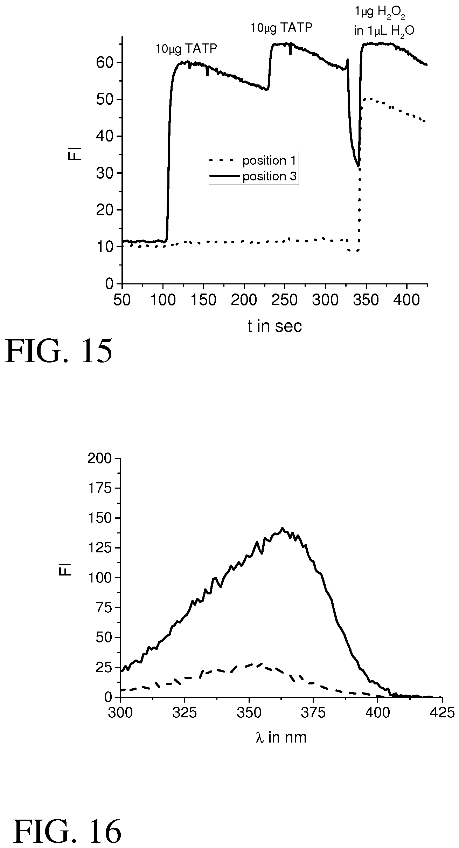

Corresponding embodiments are shown in **Figs. 16****,** **17, 18****.** Advantageously, triarylboranes in comparison to triarylamines comprise other excitation and emission wavelength ranges.

Advantageously, the target analyte is decomposed upon contact with the sulfonic acid which acts as a catalyst. Thus, at and/or within the analyte sensitive layer the organic peroxide analyte decomposes and reacts with the triarylborane dye and the reaction products are optically detected by their specific fluorescence. The corresponding reactions: catalytic decomposition and reaction with the indicator dyes take place at and/or within the carrier material. Typically, the analyte sensitive layer is fixed on a substrate, e.g. a glass or an optically transparent polymer. Therefore, the catalyst, i.e. the sulfonic acid, is dissolved with a hydrogel carrier material (e.g. a polyurethane/polyether-based hydrogel such as Hydrogel D640 from AdvanSource Biomaterials Corp.) in a mixture of an organic solvent with water and applied to the substrate, e.g. by pipetting, spotting/coating.

According to a modification of the preceding embodiment the first mixed layer further comprises a fluorescence attenuating compound which is selected from: 3,5-dinitrosalicylic acid, 3,5-dinitrobenzoic acid, 2,4-dinitrosalicylic acid, 2,4-dinitrobenzoic acid, 2,4,6-trinitrobenzoic acid, 2,4-dinitro-6-amino-benzoic acid and their esters, in particular from methylester, ethylester, phenylester and their amides, in particular from N-methylamide, N-ethylamide, N,N-dimethylamide, N,N-diethylamide, N-phenylamide, N,N-diphenylamide.

By adding at least one of the mentioned fluorescence attenuating compounds, the detection of the analyte becomes more sensitive. The fluorescence attenuating compounds decrease the fluorescence intensity of the triarylboranes in the mixed layer whereby the photomultiplier power can be increased for the peroxide induced fluorescence enhancement of the mixed layer. The fluorescence of the emerging strongly fluorescing species can more precisely be detected by using the fluorescent dye. Particularly, for the fluorescent dyes hydrophilic materials can be used as carrier material as well. Non-acidic sensor layers can also be used for the detection of TATP, DADP, and HMTD if these before are acid-catalyzed (or thermally) decomposed to hydrogen peroxide prior to interaction with the respective sensor layer. If a sulfonic acid is added to the sensor layer, and if m_{acid} > m_{carrier material}, the sensitivity of the sensor layer to hydrogen peroxide increases and the acidic sensor layers also react with TATP, DADP, and HMTD without the additional catalyst layer (second separate layer). If the peroxides are additionally decomposed with the acid catalyst (acid, hydrogel/acid, acid/TBA-salt or hydrogel/TBA-salt/acid) before reaching an acid sensor layer, the sensitivity of the acid sensor layer increases further. The presence of a catalyst is required in the event that M_{acid} < m_{carrier} material.

Thus, according to an embodiment the sensor of any of the two preceding embodiments further comprises another first mixed layer adjacent to the second separate layer such as to be located downstream of a layer arrangement comprising the first mixed layer and the separate layer, if these layers are in contact with a fluid stream which potentially is carrying an analyte. In other words, according to this embodiment a sequence of three consecutive layers results as a first mixed layer is followed by a second separate layer, and another first mixed layer along a direction of a possible analyte containing stream.

Advantageously, the first mixed layer located upstream can be used as a reference for the fluorescence signal which is measured by the other mixed layer located downstream. The second separate layer between the two of them serves as a catalyst layer. Within the catalyst layer reaction products formed upon contact of the fluid stream and in turn generate a fluorescence signal at the mixed layer downstream.

According to an embodiment the triarylborane which is selected as the molecular probe is a substance according to structure (14): 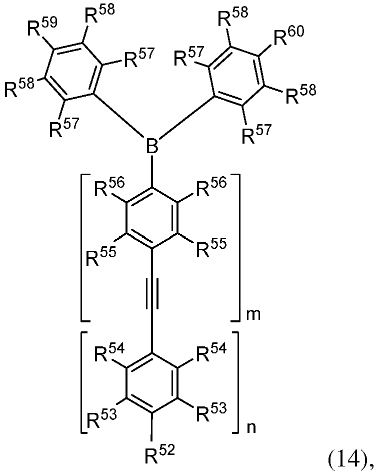 wherein a bond between the residue R⁵² and the adjacent phenyl group is selected from: a single sigma bond as shown, a phenylene group, a double bond, and a triple bond; and
- R⁵² =: B(O)₂²⁻, B(OH)(O)⁻, B(OH)₂, B(OH)₃⁻, B(OMe)₂, B(OEt)₂, B(OPr)₂, B(Oalkyl)₂, B(OPh)₂, B(Oaryl)₂ or B(OCH₂CH₂)ₓOR²)₂ wherein R² = H, Me or alkyl with x = 1, 2, 3;
- n, m =: 0, 1, 2, 3, 4; wherein n+m ≥ 1;
- R⁵³, R⁵⁶, R⁵⁷ =: H, Me, alkyl, F, Cl, Br, I, CH₂OH, CH₂O(alkyl), CH₂NMe₂, CH₂N(alkyl)₂, CH₂P(t-Bu)₂, CH₂P(alkyl)₂, OMe, OiPr, O(alkyl), O(poly)ethylene oxide;
- R⁵⁴, R⁵⁵, R⁵⁸ =: H, Me, alkyl, Ph, aryl, F, Cl, Br, I, NMe₂, N(alkyl)₂; and
- R⁵⁹, R⁶⁰ =: H, Me, alkyl, Ph, aryl, F, Cl, Br, I, CF₃, NMe₂, N(alkyl)₂, 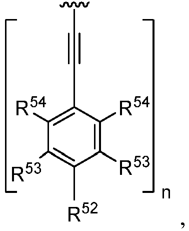
wherein n = 1, 2, 3, 4; or (according to a modification of this embodiment)
- R⁵² =: a boronic ester, respectively a cyclic boron compound, of glycol, pinacol, 1,4-butanediol, 1,5-pentanediol, tartaric acid, 2,2-dimethyl-1,3-propanediol, a cyclic boronate of methyliminodiacetic acid (MIDA), a cyclic boronamide of brenzcatechine, 1,8-diaminonaphthalene, o-phenylenediamine, or a derivative of them; wherein n, m = 0, 1, 2, 3, 4, wherein (n+m) ≥ 1;
- R⁵³, R⁵⁶, R⁵⁷ =: H, Me, alkyl, F, Cl, Br, I, CH₂OH, CH₂O(alkyl), CH₂NMe₂, CH₂N(alkyl)₂, CH₂P(t-Bu)₂, CH₂P(alkyl)₂, OMe, OiPr, O(alkyl), O(poly)ethylenoxide;
- R⁵⁴, R⁵⁵, R⁵⁸ =: H, Me, alkyl, Ph, aryl, F, Cl, Br, I, NMe₂, N(alkyl)₂; and
- R⁵⁹, R⁶⁰ =: H, I, Me, NMe₂, N(alkyl)₂, NPh₂, N(aryl)₂, ethynyl, trimethylsilylethynyl, 1-propynyl, alkynyl, phenylethynyl.

Further, the triple bond shown in structure (14) and in one of the optional residues R⁵⁹, R⁶⁰ may be replaced by a double bond, a phenylene group or a simple sigma bond.

Advantageously, the deprotonated boronic acid and the boronic ester in the acidic layer may be transformed into the boronic acid. Thus, the corresponding sensor, i.e. its first mixed layer, according to this embodiment may comprise a sulfonic acid and a boronic acid. If the salts and esters are introduced, they are hydrolyzed and/or protonated in the acidic layer to boronic acids. Advantageously, the hydrolysis and/or protonation of the boronic ester can be reduced in a hydrophobic environment. Functionalized boronic acids show low fluorescence. These modifications comprise a higher stability in a non acidic enviroment.

According to an embodiment the triarylborane is selected among a substance according to structure (14'), (14") and (14"') below: 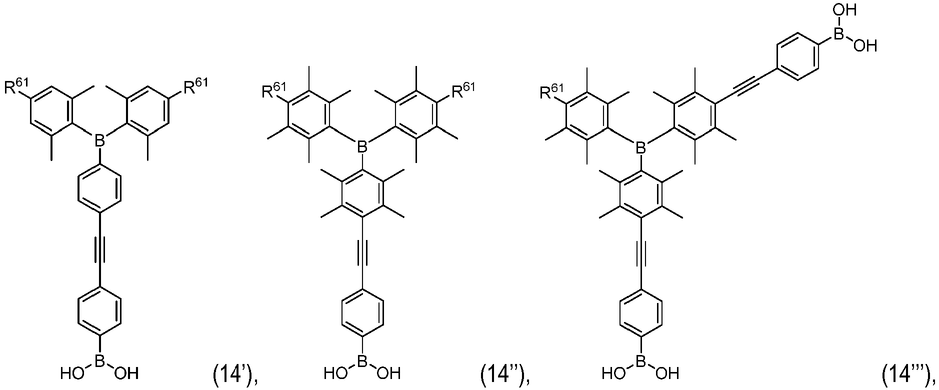 wherein R⁶¹ = H, F, Cl, Br, I, Me, alkyl, aryl, vinyl, ethynyl, alkynyl, CF₃, NMe₂, NPh₂, BMes₂.

Herein, "Mes" stands for mesityl (cf. https://www.wikidata.org/wiki/Q1898549). As to the designation of the listed residues above and in general we note that functional groups like NMe₂ can be written with or without hyphen. There the hyphen is omitted. Advantageously, these modifications comprise a higher stability, a higher selecticvity, and lower fluorescence (which allows enhancing the used photomultplier effect) and a higher fluorescence enhancement after reaction with peroxides.

According to an embodiment the molecular sensor comprising the triarylborane is selected from a substance according to structure (14*): 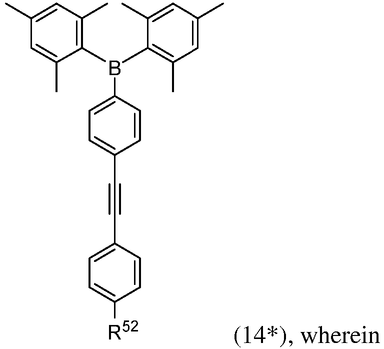
- R⁵² =: B(O)₂²⁻, B(OH)(O)⁻, B(OH)₂, B(OH)₃⁻, B(OMe)₂, B(OEt)₂, B(OPr)₂, B(Oalkyl)₂, B(OPh)₂, B(Oaryl)₂, or B(OCH₂CH₂)ₙOR²)₂, wherein R² = H, Me, or alkyl with x = 1, 2, 3;
or
- R⁵² =: boronic ester respectively cyclic boron compounds of glycol, pinacol, 1,4-butanediol, 1,5-pentanediol, tartaric acid, 2,2-dimethyl-1,3-propanediol, a cyclic boronate of methyliminodiacetic acid (MIDA), i.e. at least one of the cyclic boronates of MIDA, any of a cyclic boronamide of brenzcatechine, 1,8-diaminonaphthalene, o-phenylenediamine, or derivatives thereof.

According to a modification of the preceding embodiment, between the residue R⁵² and the adjacent phenyl group of the substance (14) and (14*), and between the B(OH)₂ and the adjacent phenyl group of substance (14'), (14"), and (14"'), a double or a triple bond or a phenylene group can be formed. Further, the triple bond shown in structure (14), (14'), (14"), (14"'), and (14*) can be replaced by a double bond, a phenylene group or a simple sigma bond.

Special advantages of these modifications comprise a higher stability, a higher selectivity, and a lower fluorescence (which allows enhancing the used photomultplier effect and a higher fluorescence enhancement after reaction with peroxides.

According to an embodiment the triarylborane is a substance according to structure (15) as indicated below:

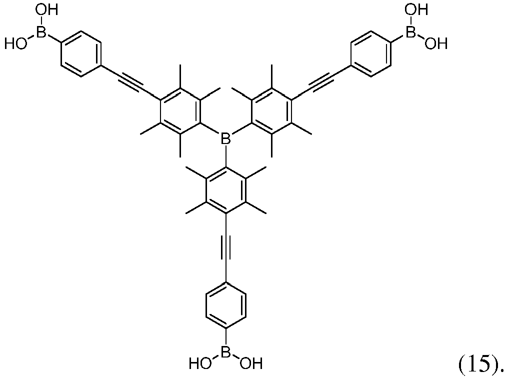

Advantageously, by the increased amount of boronic acid groups in the dye structure and in the sensor layer, the reaction rate of the dye (15) with hydrogen peroxide increases. Dye (15) has also a higher polarity that facilitates the permeability of hydrogen peroxide in to the sensor layer. The terminal boronic acid groups can be esterified using glycol, pinacol, 1,4-butanediol, 1,5-pentanediol, 2,2-dimethyl-1,3-propanediol, tartaric acid, methyliminodiacetic acid (MIDA) and can be introduced as benzamide of brenzcatechine, o-phenylenediamine, and derivatives thereof as well. If not esterified, dye (15) can also act as a cross-linker to prepare a highly porous sensor layer. Due to its larger available surface, the sensitivity of such porous sensor layer for the analytes is increased.

According to an embodiment, in the sensor comprising a triarylborane molecular sensor the sulfonic acid is selected from a substance according to structure (1), (2) and (3): 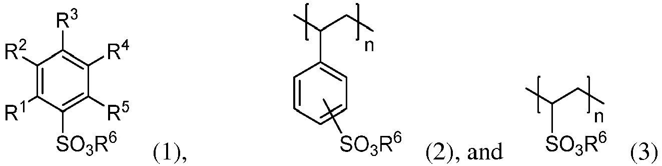
or their mixture, wherein
   - R¹, R⁵=: H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO₃⁻;
   - R², R⁴=: H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO3⁻;
   - R³ =: H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO₃⁻, (poly)ethylene oxide,
wherein n ≥ 8, particularly, 8 ≤ n ≤ 500, preferably: 150 ≤ n ≤ 400;
   or
wherein the sulfonic acid is selected from a perfluorosulfonic acid according to structure (6) and (7), or their mixture: 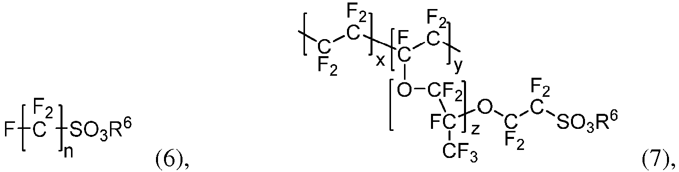 with
   - R⁶: = H, Na, K, tetrabutylammonium, tetraoctylammonium, tetraalkylammonium;
   - n: ≥ 8,
   - x: = y = 50-150, especially 75-125, and

   z= 0, 1, 2, 3.

The substance (7) and related materials are also known, e.g., as Nation^{™}, Hyflon^{™}, Aquivion^{™}, and 3M^{™}-ionomer and - advantageously - commercially available.

Further examples of suitable sulfonic acids include: octyl sulfonic acid, dodecyl sulfonic acid, octadecyl sulfonic acid, lauryl sulfonic acid, paraffin wax sulfonic acid, naphthalene sulfonic acid, lauryl cyclohexyl sulfonic acid, dodecyl benzene sulfonic acid, polyethylene sulfonic acids of various molecular weights and their salts comprising a cation selected from: a sodium, a potassium, an ammonium,
a methylammonium, a dimethylammonium, a trimethylammonium, a tetramethylammonium, a tetraethylammonium, a tetrapropylammonium, a tetrabutylammonium and/or a tetraoctylammonium cation. Furthermore, styrene maleic anhydride co-polymers bearing sulfonate groups on the rings or styrene maleic anhydride co-polymers which have been partially or fully converted to the corresponding imide sulfonates can be used in the suggested sensor.

According to an embodiment of the preceding sensor, the sulfonic acid of the sensor comprising the triarylborane molecular probe is selected from a substance according to structure (1), wherein, independently from each other,
- R¹, R⁵: = H, Me, Et, CF₃, SO₃H, CO₂H, PO₃H₂, F, Cl, Br, or I;
- R², R⁴: = H, Me, Et, CF₃, SO₃H, CO₂H, PO₃H₂, F, Cl, Br, or I; and
- R³: = H, Me, Et, CF₃, SO₃H, CO₂H, PO₃H₂, F, Cl, Br, or I.

Advantageously, the sulfonic acids are not volatile at 25-70°C and 1 atm and decompose TATP to hydrogen peroxide.

According to an embodiment the second separate layer of the sensor comprising the triarylborane is arranged adjacent to the first mixed layer and downstream with respect to a fluid stream, the fluid stream potentially containing the analyte. In other words, the second separate layer does not overlap the mixed layer, i.e. it does not cover the mixed layer and is not arranged underneath it.

Advantageously, the second separate layer, which is a catalytic layer, initiates disintegration of the analytes to the reaction products like hydrogen peroxide formed in the fluid stream after passing of the analytes without disintegration with the first mixed layer (reference layer).

According to an embodiment the second separate layer and the first mixed layer are arranged on the same substrate.

Advantageously, the corresponding microfluidic system may be compact and designed as a disposable.

According to an embodiment a second mixed layer is arranged downstream of the second separate layer of the sensor comprising the triarylborane.

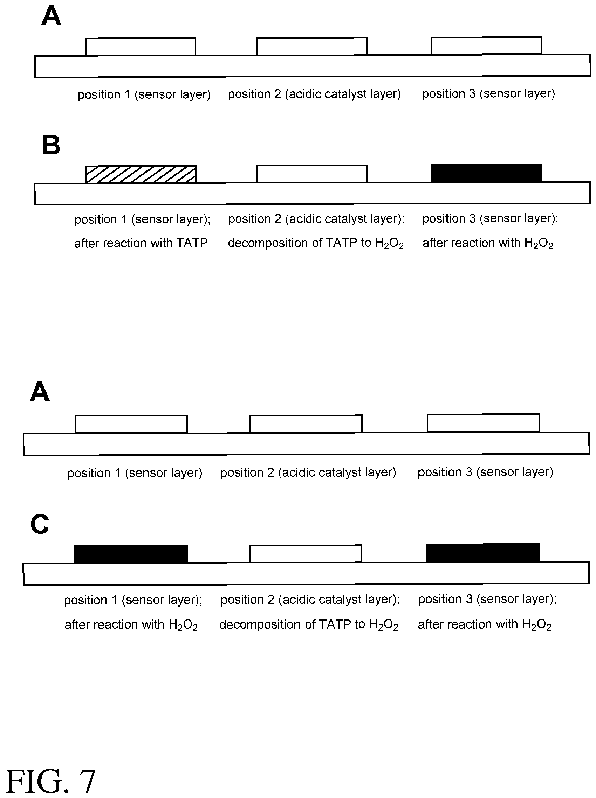

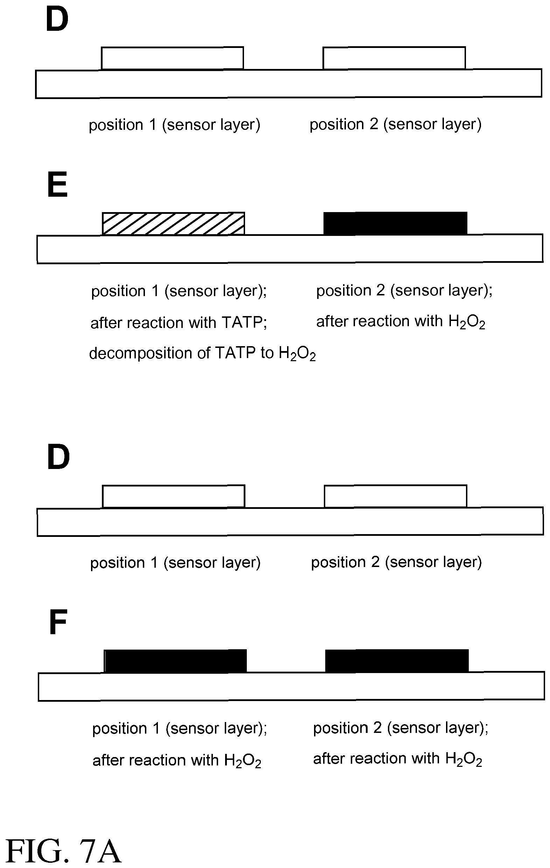

Therein "downstream" is related to a liquid stream which potentially carries the analyte, i.e. an air, a gas or a liquid stream which reaches the second separate layer only after being in fluidic contact with the first mixed layer, and correspondingly, reaches the second mixed layer only after streaming along/over the first mixed layer. Thus, the fluid first flows over the mixed layer and only then over the second separate layer and finally reaches the second mixed layer. The second mixed layer may have the same composition like the first mixed layer. If the sensor comprises a first mixed layer, a second separate layer, and a second mixed layer, e.g. as shown e.g. in **Fig. 7****,** the first mixed layer (at position 1) fulfills the function of a reference layer. It reacts with hydrogen peroxide in the fluid stream but not with TATP or DADP. Even if the reference layer (the first mixed layer) shows an increase of the fluorescence signal, the fluorescence intensity and/or its increase is higher/faster for the second mixed layer (at position 3) which indicates the presence of TATP or DADP. The second separate layer (i.e. the layer at position 2) fulfills the function of a catalyst layer. Thus, the second mixed layer (at position 3) fulfills the function of a detection layer for analytes TATP and DADP.

In a further aspect the present invention provides a method of fabricating a sensor. The method comprises:
- mixing a molecular probe, a sulfonic acid, and a hydrophilic compound; and
- forming a first mixed layer comprising the mixed molecular probe, the sulfonic acid, and the hydrophilic compound on a substrate;
wherein the molecular probe is selected from a triarylborane.

Advantageously, the method can easily be standardized to obtain uniform sensors which can be provided for routine analyses.

For instance, said fabricating may comprise:
- providing a molecular probe, a fluorescent dye, a iodinated sulfonic acid or a sulfonic acid/organo-iodine compound, and a hydrophilic compound fulfilling the function of a carrier material; wherein either the carrier material is selected such as to form a film (or a foil) or wherein a mixing ratio in a mixture of the constituents above is selected such as to form upon drying of the mixture a film or foil;
- applying on a substrate such as, e.g., a chip, a mixture of the above constituents. Optionally, the applied mixture can be dried before storage.

Advantageously, the substrate surface belongs to a microfluidic system, i.e. a lab on a chip, so after drying the chip is ready for use with the corresponding detector. Typically, the chip is fitted within a holding device in front of a detector which is sensitive in the emission range of the formed complex (see above). A light source, e.g. a LED emitting light of 365 nm is suitably arranged on the same side with respect to the chip in order to measure a backscatter fluorescence signal. Alternatively, the LED may be arranged on the opposite side of the chip and the light emitted by the light source passes through the chip, starting, e.g., from the underside (the channel side is irradiated only after the sensor layer). Therefore, if a dye is applied to the channel side, its fluorescence is absorbed if the sensor layer is exposed to hydrogen peroxide, so that less light reaches the detector. This is caused by the light absorbing species, which is generated after exposure to hydrogen peroxide and whose absorption spectra completely overlaps the emission spectra of the mentioned dye. By using, e.g., 1,5-naphthalenedisulfonic acid, which is a fluorescent compound, no other dye must be added to the sensor's mixed layer, since the absorption spectra of the light absorbing species overlaps with the emission spectra of 1,5-naphthalenedisulfonic acid. Different arrangements of the light source, the detector, the analyte stream, and the mixed layer with respect to each other will be selected depending on the circumstances (e.g. the transparency of the substrate) by the skilled person. Accordingly, a decrease in fluorescence signal intensity can be measured which depends on the concentration of the analyte. Advantageously, a microfluidic system comprising at least a channel for delivering a gaseous analyte to the sensor layer is provided. The microfluidic system can easily be prepared, e.g. by hot embossing or injection molding and subsequent deposition of a sensor layer, e.g. by drop coating. Channel width and depth, as well as the size of reservoirs (cavities/wells) can easily be designed such as to allow a lateral flow or frontal flow of a gas which carries the analyte to the sensor layer. Such microfluidic systems, i.e. *ready-to-use-lab-on-a-chip* are easily to prepare and can be stored as disposables for already existing optical equipment, e.g. a portable reader.

According to an embodiment in the method as suggested above, the hydrophilic compound and/or a mixing ratio of the molecular probe, the sulfonic acid, and the hydrophilic compound is/are selected such as to form - optionally, upon drying - a free standing film.

The free-standing film is formed after a short period (1 - 10 min) of drying under either atmospheric pressure and in a standard environment (20 °C) or under reduced pressure (~1 mbar) in the designated wells on a microfluidic gas chip into which it had been deposited, e.g. by spotting. After the free-standing film has been formed, the microfluidic gas chip is stored in an Argon-filled and light-tight aluminum container which is air-tight sealed / welded. This storage is a precautionary measure; the free-standing films are also stable under normal laboratory conditions. Herein the free standing film is able to take up water and to prevent flowing or capillary movement in a microfluidic system which is adapted to guide a gas stream over consecutively arranged layers. In particular, the hydrophilic compound may absorb water. If the first mixed layer comprises a salt of a sulfonic acid, e.g. a tetraalkylammonium sulfonate, it becomes even hygroscopic. Advantageously, the mixture of the molecular probe and the hydrophilic compound (and any salt) is adapted such as to prevent any movement of the sensor in a microfluidic system. Advantageous salts comprise cations selected from: tetraoctylammonium, tetrahexylammonium, tetrabutylammonium, tetraethylammonium, tetramethylammonium, tetraalkylammonium, trialkylammonium, dialkylammonium, alkylammonium, ammonium, alkylanilinium, dialkylanilinium, Li⁺, Na⁺ and K⁺. For the preparation of the catalyst the sterically hindered tetraalkylammonium salts are preferred because they are hygroscopic and prevent the crystallization of the acid.

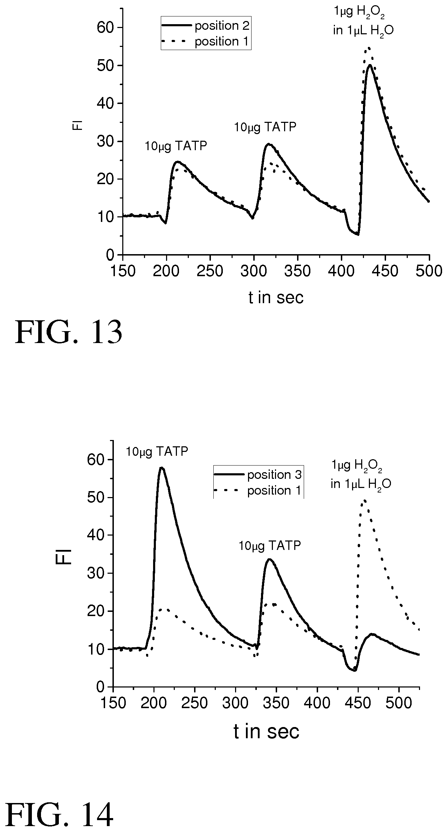

The proposed sulfonic acids in the catalyst layer decompose the peroxidic explosives into hydrogen peroxide. The peroxides are first fed (i.e. a gas stream containing the analytes) onto the first sensor layer containing the first mixed layer, comprising the hydrophilic carrier material, a sulfonic acid and, e.g. a triarylborane dye (m_{carrier material}/m_{acid} >1), which shows a low reactivity with TATP and DADP. According to an embodiment, the ratio 1 < (m_{carrier material}/m_{acid}) < 5, e.g. (m_{carrier material}/m_{acid}) = 1.1 or 3.3. Afterwards, the peroxidic explosives in the gas stream are fed to the acidic catalyst layer (i.e. to the "second separate layer", herein also designed as "catalytic layer") where they decompose to hydrogen peroxide and then onto another mixed layer (m_{carrier material}/m_{acid} >1) where the mass fraction of the carrier material is also greater than the mass fraction of the acid fraction (m_{carrier material}/m_{acid} >1). TATP and DADP show with the second sensor layer a high reactivity. Typically, both mixed layers have the same composition. Thus, the first sensor layer acts as a reference layer to distinguish TATP and DADP from hydrogen peroxide (cf. **Fig. 7**). Another way of decomposing peroxidic explosives to hydrogen peroxide and detecting it on the same layer is to apply the three mentioned components, i.e. the hydrophilic compound as a carrier material, a sulfonic acid and a triarylborane dye, as a mixed layer to the substrate, where the mass fraction (m) of the acid is greater than that of the carrier material (m_{carrier material}/m_{acid} <1). The peroxidic explosives are thus decomposed directly on/in the first mixed layer and are simultaneously detected. However, the disadvantage of the latter method is that after the reaction of the first mixed layer with a peroxide or after the associated fluorescence increase, the fluorescence intensity of this layer rapidly decreases again because of its high acid concentration **(****Figs. 7A** and **13**).

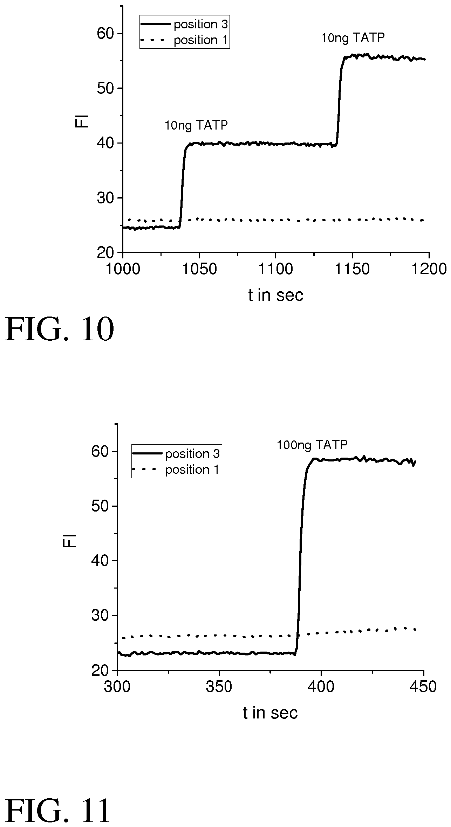

If, on the other hand, less acid is used in relation to the hydrogel, the fluorescence signal, which was amplified after the reaction of the sensor layer with peroxides, remains constant over a longer period of time (**Figs. 10, 11****,** **12** and **12A**). In addition to the catalytic decomposition of peroxidic explosives into hydrogen peroxide with the acid in a catalyst layer, i.e. in the so called "second separate layer", the acid in the first mixed layer layer performs two other tasks. Due to the hygroscopicity of the sulfonic acid(s), the water absorption capacity of the mixed layer increases which improves the hydrogen peroxide absorption capacity. The second task of the acid is to decrease the fluorescence intensity of the triarylborane and increase the sensitivity of the sensor layer.

While the pure hydrogels are difficult for the analytes to penetrate or the analyte absorption capacity is low, the acid hydrogels can absorb or dissolve the analytes easily. The acid in the sensor layer also has the task of extinguishing the fluorescence of the dye in the hydrogel or reducing the intensity of the baseline.

According to the fabrication method of the present invention the molecular probe is selected from a triarylborane, the method further comprising:
- forming a second separate layer adjacent to the first mixed layer,

wherein the second separate layer comprises:
   - a sulfonic acid, or
   - a sulfonic acid and a salt of the sulfonic acid, or
   - a sulfonic acid and the hydrophilic compound, or
   - a sulfonic acid, a salt of the sulfonic acid and a hydrophilic compound;
wherein the salt of the sulfonic acid comprises a cation selected from: a sodium, a potassium, an ammonium, a methylammonium, a dimethylammonium, a trimethylammonium, a tetramethylammonium, a tetraethylammonium, a tetrapropylammonium, a tetrabutylammonium and/or a tetraoctylammonium cation; and wherein the second separate layer does not overlap with the first mixed layer. In other words, between two identical mixed layers a second separate layer may be placed. This "second separate layer" fulfills the function of a catalyst layer. It is called second separate layer in contrast to the first separate layer, which merely comprises a layer comprising a fluorescent dye (see above).

According to an embodiment of the preceding fabrication method the second separate layer in the second depositing step further comprises a fluorescence attenuating compound which is selected from: 3,5-dinitrosalicylic acid, 3,5-dinitrobenzoic acid, 2,4-dinitrosalicylic acid, 2,4-dinitrobenzoic acid, 2,4,6-trinitrobenzoic acid, 2,4-dinitro-6-amino-benzoic acid and their esters, in particular from methylester, ethylester, phenylester and their amides, in particular from N-methylamide, N-ethylamide, N,N-dimethylamide, N,N-diethylamide, N-phenylamide, N,N-diphenylamide.

Advantageously the fluorescence attenuating compound enhances the sensitivity of the sensor for the peroxide analytes.

In another aspect the present invention provides a method for selective detection of an analyte, selected from triacetone triperoxide, diacetone diperoxide, hexamethylene triperoxide diamine and hydrogen peroxide is suggested. The method comprises:
- providing at least one sensor according to the present invention;
- directing a fluid stream comprising the vaporized or gaseous analyte on at least one layer of the sensor, wherein the fluid is preferably air or an inert gas of a temperature within a range from 15-200 °C, preferably from 50-170 °C;
- exposing the at least one layer to an excitation light, thus exciting a fluorescent compound which is formed within or at the at least one layer upon interaction with the analyte;
- detecting an intensity of a fluorescence; and
- detecting at least one of the analytes qualitatively and/or quantitatively by measuring an enhancement or a decline of the intensity.

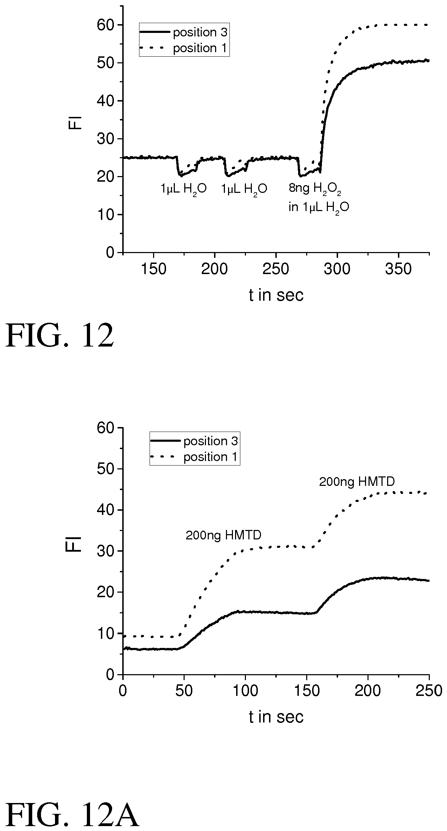

Advantageously, the suggested method allows for sensitive detection of TATP (lower detection limit / LDL = 10 ng), HMTD (LDL = 200 ng). As to the lowest detection limit of TATP and hydrogen peroxide, **Figs. 10** and **12** indicate that quantities as low as 10 ng and 8 ng could be detected, respectively. **Fig. 11** illustrates the detection of 100 ng TATP, while **Fig. 12A** illustrates the repeated detection of 200 ng HMTD. **Fig. 13** illustrates the repeated detection of 10 µg TATP and 1 µg hydrogen peroxide without a catalyst layer, while **Fig. 14** illustrates the repeated detection of 10 µg TATP and 1 µg hydrogen peroxide with a catalyst layer. **Fig. 15** illustrates the repeated detection of 10 µg TATP and 1 µg hydrogen peroxide with a catalyst layer.

According to an embodiment in the suggested method detecting the intensity comprises measuring an attenuation by absorption of at least a part of an excitation light and/or by absorption of at least a part of an emission light, i.e. a fluorescence within or by at least one layer of the sensor.

Advantageously, the sensor may easily be adopted for different concentration ranges of the analytes. Therefore, e.g., different sets of microfluidic systems or a single microfluidic system comprising different channels with different sensitivities may be provided. Such system is advantageously adapted to cover all relevant concentration ranges of the mentioned analytes.

According to an embodiment the provided sensor comprises a first mixed layer as described in any of the related embodiments above (corresponding to claims 1-28) and a fluorescent dye as described in any of the related embodiments above (corresponding to claims 14 through 19, in particular in claims 14, 15, and 16).

In other words, the sensor used in the suggested method according to the above embodiment (aspect 1) comprises a first mixed layer in combination with a fluorescent dye, wherein the fluorescent dye comprises an absorption maximum within a range of 340-450 nm. According to the second aspect of the invention, the appearance of a new fluorescence band after interaction of the decomposition products with the triarylborane dye is quantified according to specific fluorescence intensity by exciting within the wavelength range 330-390 nm, e.g. 350-375 nm, and measuring the fluorescence intensity within the wavelength range 400-500 nm, e.g. 410-450 nm.

According to an embodiment said sensor of said method further comprises a second separate layer as described in any of the related embodiments (corresponding to claims 20-31) which is arranged downstream of the first mixed layer, wherein triacetone triperoxide is detected by a fluorescence attenuation at the first mixed layer. Thus, the method comprises guiding a fluid stream, potentially containing at least one of the indicated analytes in fluidic contact with consecutively a first mixed layer, a second separate layer, and another first mixed layer and measuring a fluorescence signal of at least one of the mixed layers, or - preferably - calculating a ratio between the fluorescence intensities or attenuation values detected at the first mixed layer upstream and at the first mixed layer downstream.

Due to the use of a first mixed layer upstream, followed downstream by a catalyst layer and downstream again the first mixed layer a differentiation between hydrogen peroxide and TATP is highly efficient. Hydrogen peroxide reacts with the first mixed layer and the first mixed layer downstream. Whereas TATP does not react with the first mixed layer, but due to the catalyst layer, TATP is decomposed to hydrogen peroxide using acids. Therefore, TATP can be indirectly detected by the first mixed layer downstream. This sensor layer is resistant to storage at 70 °C. After storing at 70 °C for 12 days, 10 ng TATP can still be selectively detected.

Advantageously, the sensor according to the second aspect does not generate any signal in response to benzoyl peroxide, a substance extensively used in cosmetic products and dermatological ointments which might cause false positive signals. Therefore, the corresponding detection method is quite rugged and suitable for application even by an untrained person.

As to the experimental conditions in the detection method above, the step of directing a fluid stream, the temperature at the inlet of the detector is 150-175 °C (depending on a current measurement mode) and the chip is heated to 50-70 °C, so the temperature range of the chip should be within 40-100 °C. The step comprising "detection of an intensity", comprises typically measuring a fluorescence intensity over a time interval of approximately 10 s (typically a fluorescence decrease, i.e. dim-down is observed for an acquisition time of below 10 seconds). Thus, the measuring may comprise a step of detecting a dim-down of the fluorescence signal from the used dye.

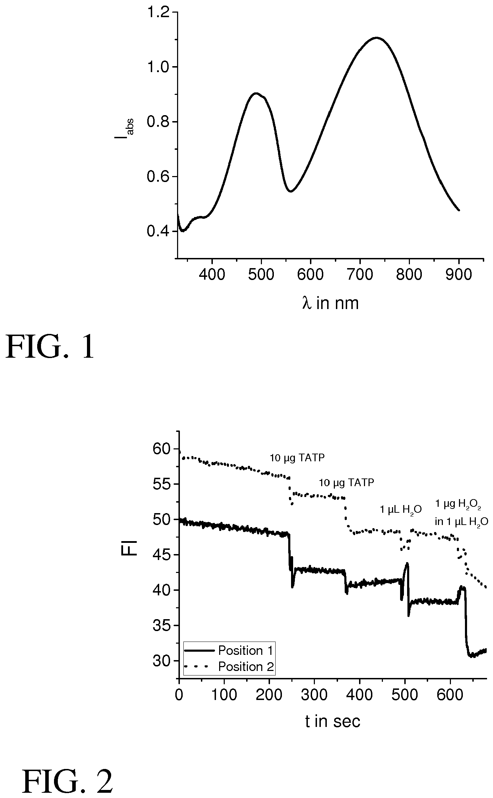

In other words: Upon exposure to hydrogen peroxide or TATP, DADP, HMTD, a reaction in the sensor layer is initiated, which leads to a formation of an oxidised triarylamine species, which shows a broad absorption spectrum from 400-550 nm and 600-850 nm. This analyte-specific light absorbing species absorbs the fluorescence of the used dye, such that a decrease in fluorescence can be measured. Absorption spectra of triphenylamine in the sensor layer after exposure to hydrogen peroxide or TATP is shown in **Fig. 1****.**

In yet other words: According to an embodiment the sensor after or while being in contact with the analyte containing gas is exposed to an excitation light (which is emitted, e.g., by a suitable LED). The excitation light is directed through or on the sensor, thus exciting a fluorescent compound whose fluorescence is absorbed by a dye present in or adjacent to a so called "first mixed layer" after reaction with the analyte. Throughout the measurement said fluorescent compound, i.e. the dye, is excited by the excitation light and thus yields a base line with the detector. Said base line serves as a starting line to measure fluorescence quenching (= attenuation = dim-down). If the analyte upon contact with the sensor layer is converted, the fluorescence of the dye is (at least partially) absorbed by the first mixed layer and a decrease of fluorescence intensity can thus be detected. However, the dye itself remains unaffected within the sensor layer or adjacent thereto, as explained above.

According to an embodiment a molecular probe for detecting an analyte selected from triacetone triperoxide, diacetone diperoxide, hexamethylene triperoxide diamine, and hydrogen peroxide by means of a fluorescence signal which generated in response to an excitation in the wavelength range between 340-370 nm is suggested. The suggested molecular probe is a triaryl borane dye according to formula (14*): 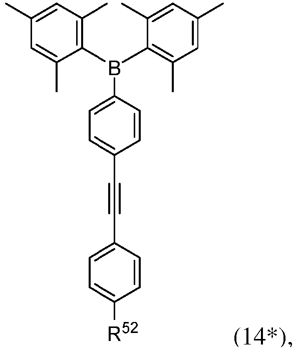 wherein
- R⁵² =: B(O)₂²⁻, B(OH)(O)⁻, B(OH)₂, B(OH)₃⁻, B(OMe)₂, B(OEt)₂, B(OPr)₂, B(Oalkyl)₂, B(OPh)₂, B(Oaryl)₂, or B(OCH₂CH₂)ₙOR²)₂, wherein R² = H, Me, or alkyl with x = 1, 2, 3;
or
- R⁵² =: boronic ester respectively cyclic boron compounds of glycol, pinacol, 1,4-butanediol, 1,5-pentanediol, tartaric acid, 2,2-dimethyl-1,3-propanediol, a cyclic boronate of methyliminodiacetic acid (MIDA) i.e. at least one of the cyclic boronates of MIDA, a cyclic boronamide of brenzcatechine, 1,8-diaminonaphthalene, o-phenylenediamine, or derivatives thereof.

Advantageously, said dye can easily be prepared, is stable in the presence of strong acids, such as the sulfonic acids described herein, and can be stored more than 6 months e.g. in an ethanol water mixture. The dye in the mixed layer generates a bright fluorescence signal with the analytes.

According to another embodiment the suggested molecular probe is a triarylborane according to structure (15) as indicated below:

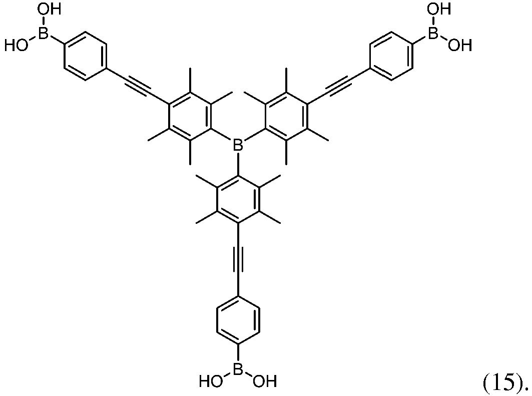

Advantageously, the boronic acid groups form easily boronic esters, respectively, cyclic boron compounds with glycol, pinacol, 1,4-butanediol, 1,5-pentanediol, tartaric acid, 2,2-dimethyl-1,3-propanediol, and methyliminodiacetic acid (MIDA), which are highly fluorescent. This molecular probe is stable as well in the presence of strong acids such as the sulfonic acids described herein and generates bright fluorescence signals in response to the analytes.

Each embodiment described above may be combined with any other embodiment or embodiments unless clearly indicated to the contrary.

### BRIEF DESCRIPTION OF THE DRAWINGS

A full and enabling disclosure of the present invention, including the best mode thereof, to one of ordinary skill in the art, is set forth more particularly in the remainder of the description, including reference to the accompanying figures.

As a comparative example,
**Figure 1** shows the absorption spectra of a sensor, comprising a first mixed layer with triphenylamine, 1,5-naphthalenedisulfonic acid, 4-iodobenzenesulfonic acid in HD1 after exposure to hydrogen peroxide.
**Figure 2** shows results of the fluorescence intensity measurement of sensors, comprising each a first mixed layer which were prepared according to the description of comparative example 1. On two fluorescence indicators were applied: twice 10 µg TATP, 1 µL water and 1 µg hydrogen peroxide in 1 µL water. For the measurement of TATP 10 mg TATP were dissolved in 10 mL acetone. 10 µL of the samples were applied every 30 s on the sensor layer and were measured each after 2 min waiting time.

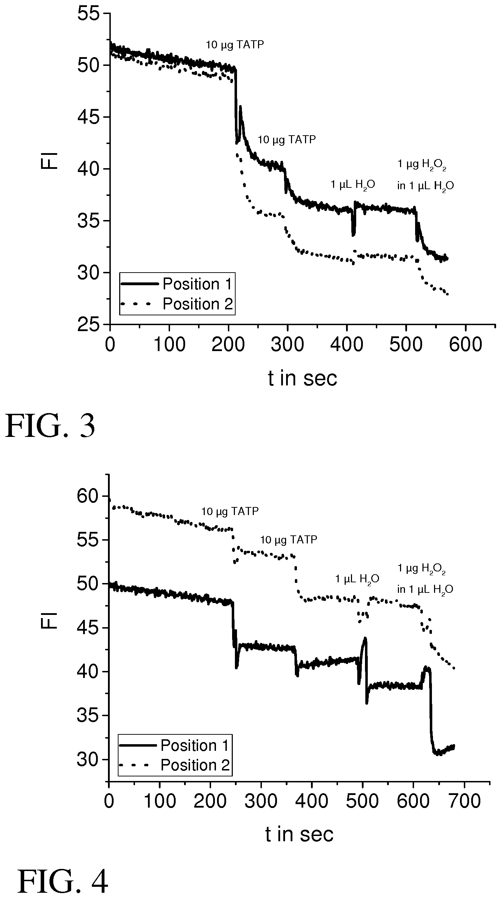

**Figure 3** shows results of the fluorescence intensity measurement of first mixed layers on glass which were prepared according to the description of comparative example 2. With two fluorescence indicators were detected: twice 10 µg TATP, 1 µL water, and 1 µg hydrogen peroxide in 1 µL water. The samples were applied every 30 s on the sensor layers and were measured each after 2 min waiting time.
**Figure 4** shows results of the fluorescence intensity measurement of first mixed layers on a Teflon foil which were prepared according to the description of comparative example 3. With two separate fluorescence indicators were detected: twice 10 µg TATP, 1 µL water, and 1 µg hydrogen peroxide in 1 µL water. The samples were applied every 30 s on the sensor layer and were measured each after 2 min waiting time.

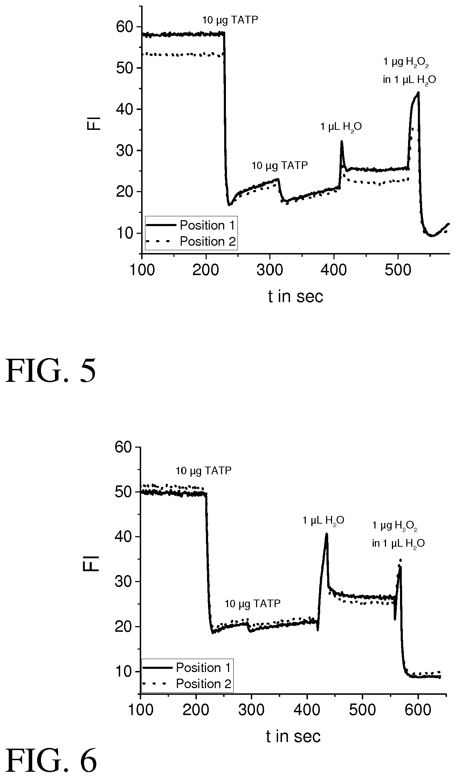

**Figure 5** shows results of the fluorescence intensity measurement of first mixed layers on a Teflon foil which were prepared according to the description of comparative example 4. With two separate fluorescence indicators were detected: twice 10 pg TATP, 1 µL water, and 1 µg hydrogen peroxide in 1 µL water. The samples were applied every 30 s on the respective sensor and were measured each after 2 min waiting time.

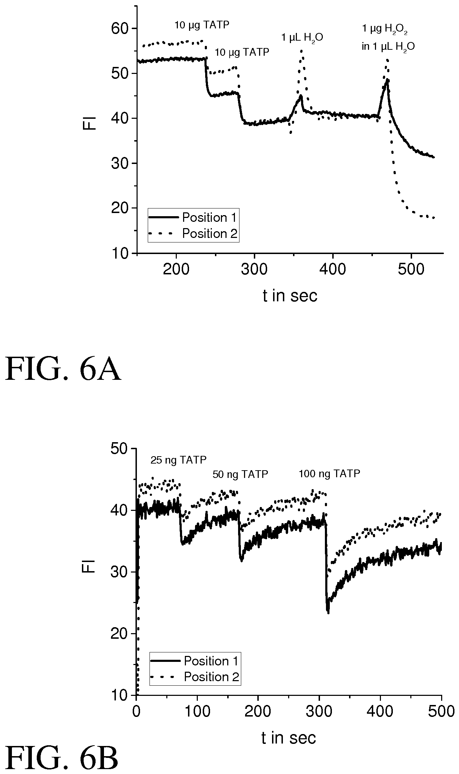

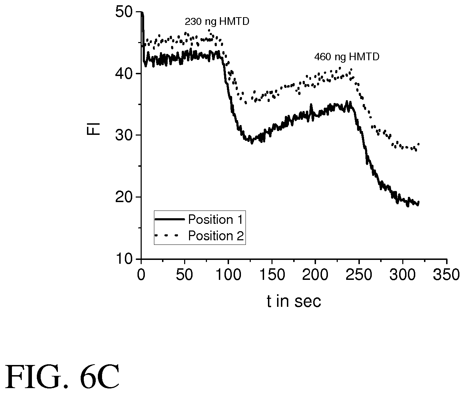

**Figure 6** shows corresponding results of the fluorescence intensity measurement of first mixed layers on a Teflon foil which were prepared according to the description of comparative example 5. In this measurement, the metal surface of the sensor head with sensors 1 and 2 was coated with a dye in polystyrene.
**Figures 6A-6C** show the corresponding results of the fluorescence intensity measurements of first mixed layers on glass which were prepared according to the description of comparative example 6. As apparent from **Fig. 6A****,** signal regeneration takes some time for a high quantity of analyte (10 µg TATP), whereas return to a stable signal is much faster for clearly lower quantities of analytes (cf. **Fig. 6B** and **Fig. 6C** for TATP and HMTD in ng-range, respectively).
**Figure** 7 shows a sequence of adjacent layers which is suitable to discriminate between hydrogen peroxide and the organic peroxides TATP if the analyte carrying fluid (gas/air) stream is flowing from the left to the right. The positions 1 and 3 comprise each a first mixed layer and the layer at position 2 is a second separate layer, i.e. an acidic catalyst layer. The scheme marked (A) shows the sensor chip before reaction with peroxides, the scheme marked (B) shows the chip after reaction with TATP and (C) illustrates the fluorescence picture after reaction with hydrogen peroxide. Strong fluorescence enhancement (from white to black); weak fluorescence enhancement (from white to hashed); no fluorescence enhancement (white). The (peroxide containing) fluid (e.g. airflow) is directed at first to position 1, than to position 2 and to position 3.
**Figure 7A** shows a sequence of adjacent layers which is suitable to discriminate between hydrogen peroxide and the organic peroxide TATP if the analyte carrying fluid (gas) stream is flowing from left to right. The positions 1 and 2 comprise each a first mixed layer. Sensor before reaction with peroxides (D), after reaction with TATP (E) and after reaction with hydrogen peroxide (F). Strong fluorescence enhancement (from white to black); fluorescence enhancement (from white to hashed); no fluorescence enhancement (white). The (peroxide containing) airflow is directed at first to position 1 and then to position 2.

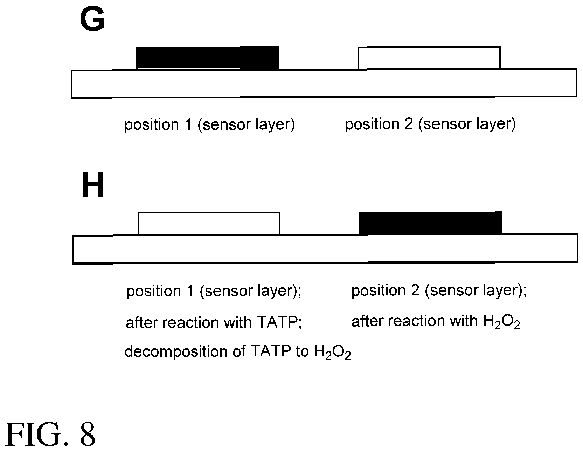

**Figure 8** shows a combination of different layers: At position 1 is a first mixed layer as described according to the first aspect and at position 2 is a first mixed layer according to the second aspect (i.e. with a triarylborane instead of a triarylamine as the molecular probe) with low acid concentration (m_{carrier material}/m_{acid} > 1) described above, before and after contact with TATP and hydrogen peroxide. Position 1 contains the first mixed layer according to aspect 1. Position 2 contains the first mixed layer according to aspect 2. Sensor chip before reaction with peroxides (G), after reaction with TATP (H). Position 1 shows a fluorescence quenching and position 2 a fluorescence enhancement. Fluorescence enhancement (from white to black); fluorescence quenching (from black to white). The (peroxide containing) airflow is directed at first to position 1 and then to position 2.

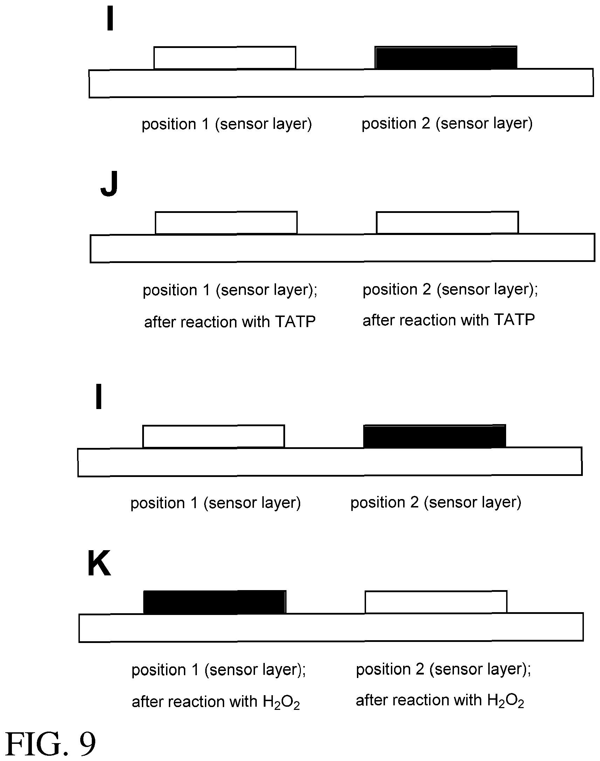

**Figure 9** shows the reversal of the arrangement of **Fig. 8****,** i.e. a first mixed layer combination according to the second aspect at position 1 with low acid concentration (m_{carrier material}/m_{acid} >>1) and a sensor/catalyst layer according to the first aspect at position 2. The sensor chip before reaction with peroxides (I), after reaction with TATP (1) and after reaction with hydrogen peroxide (K). Strong fluorescence enhancement (from white to black); fluorescence quenching (from black to white).
**Figure 10** and **Figure 11** show on two different charts measurement signals (fluorescence light intensity - FI) for different quantities of TATP (10 ng TATP - **Fig. 10** and 100 ng - **Fig. 11**) on Teflon as measured with a combination of two "first mixed layers" on position 1 and 3 and a "second separate layer" (i.e. a catalyst layer) on position 2 of a COC-type lab-on-chip according to **Fig. 7****.** The analyte sensitive layers ("first mixed layers") and the catalyst layer ("second separate layer") are prepared according to example 7 and, respectively.
**Figure 12** shows measurement signals (fluorescence light intensity - FI) for 1 µL water and 8 ng hydrogen peroxide in 1 µL water as measured with two "first mixed layers" on position 1 and 3 and a "second separate layer" on position 2 of a COC-type lab-on-chip according to **Fig. 7****.** The analyte sensitive layers ("first mixed layers") and the catalyst layer ("second separate layer") are prepared according to example 7 and 9b.
**Figure 12A** shows measurement signals (fluorescence light intensity - FI) for 200 ng HMTD on Teflon as measured with two "first mixed layers" on position 1 and 3 and a "second separate layer" on position 2 of a COC-type lab-on-chip according to **Fig. 7****.** The analyte sensitive layers ("first mixed layers") and the catalyst layer ("second separate layer") are prepared according to example 7 and 9b.
**Figure 13** shows measurement signals (fluorescence light intensity - FI) of 10 µg TATP and 1 µg hydrogen peroxide in 1 µL water on Teflon as measured with two "first mixed layers" on position 1 and 2 on glass according to **Fig. 7A****.** The analyte sensitive layers ("first mixed layers") are prepared according to example 10.
**Figure 14** shows measurement signals (fluorescence light intensity - FI) of 10 µg TATP and 1 µg hydrogen peroxide in 1 µL water as measured with two "first mixed layers" on position 1 and 3 and a "second separate layer" on position 2 on glass according to **Fig. 7****.** The analyte sensitive layers ("first mixed layers") and the catalyst layer ("second separate layer") are prepared according to example 10 and 8.
**Figure 15** shows measurement signals (fluorescence light intensity - FI) of 10 µg TATP and 1 µg hydrogen peroxide in 1 µL water as measured with two "first mixed layers" on position 1 and 3 and a "second separate layer" on position 2 on glass according to **Fig. 7****.** The analyte sensitive layers ("first mixed layers") and the catalyst layer ("second separate layer") are prepared according to example 11 and 9a.
**Figure 16** shows fluorescence excitation spectra of an analyte sensitive layer with a triarylborane dye according to structure (14') with R⁶¹ = Me (or according to structure (14*) with R⁵² = B(OH)₂) before (dashed) and after (solid line) the presence of hydrogen peroxide in the carrier gas. The peroxide sensitive layer was prepared according to the following procedure: Solution 1 = 576 mg Hydromed^{™}-D7 (referred to as HD7 hereinafter) + 6404 mg ethanol + 754 mg water; solution 2 = 7 mg 4-bromobenzenesulfonic acid monohydrate + 107 mg solution 1 + 152 mg ethanol + 17 mg water + 0.7 mg the triarylborane dye according to structure (14') with R⁶¹ = Me; solution 3 = 28 mg solution 2 + 17.6 µg 3,5-dinitrosalicylic acid; 5 µL of the solution 3 is drop-coated onto the COC-substrate.

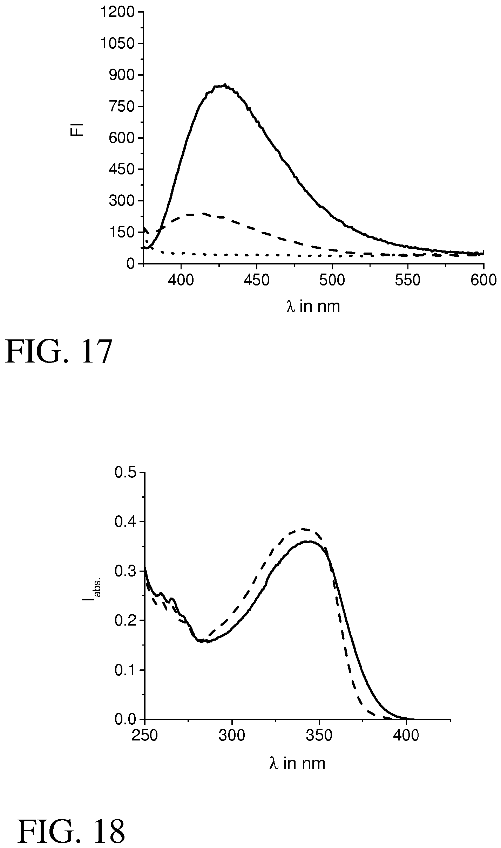

**Figure 17** shows the emission spectra of the analyte sensitive layer with a triarylborane dye according to structure (14') with R⁶¹ = Me before (dashed) and after (solid line) the presence of hydrogen peroxide in the carrier gas; background (dotted). The analyte sensitive layer ("first mixed layer") was prepared as described above with respect to **Fig. 16****.**
**Figure 18** shows the absorption spectra of the analyte sensitive layer ("first mixed layer") with a triarylborane dye according to structure (14') with R⁶¹ = Me (dashed) and after (solid line) the presence of hydrogen peroxide in the carrier gas. The analyte sensitive layer was prepared as described above with respect to **Fig. 16****.**
In the following detailed description, reference is made to the accompanying figures, which form a part hereof, and in which are shown by way of illustration specific embodiments and features of the invention. It is to be understood that other embodiments may be utilized and structural or logical changes may be made without departing from the scope of the present invention. The following detailed description, therefore, is not to be taken in a limiting sense, and the scope of the present invention is defined by the appended claims.

### DETAILED DESCRIPTION

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

As used in this specification (above and below) and claims, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

As used in this specification (above and below) and claims, the word "substantially" when used with a numerical value is to be understood as encompassing a deviation from the indicated value within a range which is typical for the method or apparatus used for measuring the value. In particular, the word "substantially" indicates including a deviation from the indicated value by as much as ± 5%, i.e. a range. When used with an adjective or adverb the word "substantially" is intended to enhance the scope of the particular characteristic; e.g., substantially planar is intended to mean planar, nearly planar and/or exhibiting characteristics associated with a planar element.

As used in this specification (above and below) and claims, the terms "fluorescence measurement", "fluorescent dye", and any related thereto term is to be understood as comprising an optical property, in particular a luminescence or its detection, e.g., an excitation wavelength, an emission wavelength, a fluorescence intensity, a fluorescence quantum yield, a fluorescence life time or decay, a fluorescence ratio, or the like and/or its detection.

The technical object of the described embodiments is to provide necessary elements of a device, the device itself, and its use, i.e. a method of analysis, for detecting minute amounts of peroxide explosives and products of their decomposition wherever they may occur: from soil samples, from wipe samples and from fluid extracts of different matrices. Therein, the detection is accomplished in fluid streams comprising gaseous or vaporized residues of the peroxide explosives. The method and device allow discriminating between residues of hydrogen peroxide and peroxidic explosives such as TATP (triacetone triperoxide), DADP (diacetone diperoxide), and HMTD (hexamethylene triperoxide diamine). Accordingly, the device and method shall allow detection of these substances in nano- and picogram quantities.

### EXAMPLES

### Example 1

A first mixed layer was prepared on a glass substrate by using the ether-based hydrophilic polyurethane Hydromed^{™}-D4, designated as HD4 thereinafter, the sulfonic acid 4-iodobenzenesulfonic acid and the arylamine N,N,N',N'-tetraphenylbenzidine according to the earlier mentioned structure (11) where R³², R³³, R³⁴, R³⁵, R³⁶, R ³⁷, R³⁸, R ³⁹, R⁴⁰, R⁴¹, R⁴², R⁴³, R⁴⁴, R⁴⁵, R⁴⁶, R⁴⁷, R⁴⁸, R⁴⁹, R⁵⁰ and R⁵¹ = H as follows:
1. 2 g HD4 were dissolved in 22 g EtOH and 2.5 g H₂O (solution A).
2. 3.0 mg of the arylamine were dissolved in 75 µL EtOH and 75 µL ethyl acetate (solution B).
3. 22.3 mg 4-iodobenzenesulfonic acid was dissolved in 50 µL EtOH and 50 µL ethyl acetate (solution C).
4. A mixture of 1 µL of solution A, 11 µL of solution B and 5 µL of solution C were put for 30 min at 60 °C in an ultrasonic bath.
5. 0.5 µL of the resulting mixture were applied onto the chosen substrate by drop-coating. After drying under reduced pressure, the first mixed layer was stored in the dark excluding any moisture.

Other names of the mentioned substance N,N,N',N'-tetraphenylbenzidine are: Benzidine, N,N,N',N'-tetraphenyl- (6CI,7CI,8CI); [1,1'-Biphenyl]-4,4'-diamine, N,N,N',N'-tetraphenyl- (9CI); N,N,N',N'-Tetraphenyl-[1,1'-biphenyl]-4,4'-diamine; N,N,N',N'-Tetraphenylbenzidine; N,N,N',N'-Tetraphenylbiphenyl-4,4'-diamine.

For the detection of TATP 10 µg of TATP were dissolved in 10 mL acetone. 10 µL samples of this solution were placed at intervals of 30 s on a Teflon foil each and measured after 2 min waiting time.

### Example 2

Further, another first mixed layer was prepared on a glass substrate by using the ether-based hydrophilic polyurethane Hydromed^{™}-D7, designated as HD7 thereinafter, the sulfonic acid 4-iodobenzenesulfonic acid and the arylamine N,N'-bis(3-methylphenyl)*-N,N'-*diphenylbenzidine according to the earlier mentioned structure (11) with R³², R³⁴, R³⁵, R³⁶, R³⁷ , R ³⁸ , R³⁹, R⁴⁰, R⁴¹, R⁴², R⁴³, R⁴⁴, R⁴⁵, R⁴⁶, R⁴⁷, R⁴⁸, R⁴⁹ and R⁵¹ are identical = H; and the residues R³³ and R⁵⁰ are identical = Me as described below.

Particularly, the first mixed layer , i.e. the fluorescence indicator according to example 2 was prepared as follows:
1. 2 g HD7 were dissolved in 22 g EtOH and 2.5 g H₂O (solution A).
2. 3.0 mg of the arylamine according to structure (11) mentioned above were dissolved in 75 µL EtOH and 75 µL ethyl acetate (solution B).
3. 22.3 mg 4-iodobenzenesulfonic acid were dissolved in 50 µL EtOH and 50 µL ethyl acetate (solution C).
4. A mixture of 1 µL of solution A, 11 µL of solution B and 5 µL of solution C were put for 30 min at 60 °C in an ultrasonic bath.
5. 0.5 µL of the resulting mixture were applied onto the chosen substrate by drop-coating.
6. After drying under reduced pressure, the first mixed layer was stored in the dark excluding any moisture. Detection of TATP was tested as described above.

### Example 3

Further, another first mixed layer was prepared on a glass substrate by using the ether-based hydrophilic polyurethane Hydromed^{™}-D4, designated as HD4 thereinafter, the sulfonic acids, 4-iodobenzenesulfonic acid and 1,5-naphthalenedisulfonic acid, and triphenylamine as described below:
1. 2 g HD4 were dissolved in 22 g EtOH and 2.5 g H₂O (solution A).
2. 5.6 mg triphenylamine were dissolved in 75 µL EtOH and 75 µL ethyl acetate (solution B).
3. 6.9 mg 1,5-naphthalenedisulfonic acid were dissolved in 50 µL EtOH and 50 µL ethyl acetate (solution C).
4. 22.3 mg 4-iodobenzenesulfonic acid were dissolved in 50 µL EtOH and 50 µL ethyl acetate (solution D).
5. A mixture of 2 µL solution A, 3 µL solution B, 15 µL solution C and 5 µL solution D were ultrasonicated for 30 min at 60 °C.
6. 0.5 µL of the mixture were applied to the substrate by drop-coating.
7. After drying under reduced pressure, the sensor was stored under exclusion of light and moisture.

### Example 4

Further, another first mixed layer was prepared on a glass substrate by using the ether-based hydrophilic polyurethane Hydromed^{™}-D7, designated as HD7 thereinafter, the sulfonic acids, 4-iodobenzenesulfonic acid and 1,5-naphthalenedisulfonic acid, and triphenylamine as described below:
1. 2 g HD7 were dissolved in 22 g EtOH and 2.5 g H₂O (solution A).
2. 5.6 mg triphenylamine were dissolved in 75 µL EtOH and 75 µL ethyl acetate (solution B).
3. 6.9 mg 1,5-naphthalenedisulfonic acid were dissolved in 50 µL EtOH and 50 µL ethyl acetate (solution C).
4. 22.3 mg 4-iodobenzenesulfonic acid were dissolved in 50 µL EtOH and 50 µL ethyl acetate (solution D).
5. A mixture of 2 µL of solution A, 3 µL solution B, 15 µL solution C and 5 µL solution D were ultrasonicated for 30 min at 60 °C.
6. 0.5 µL of the mixture were applied to the substrate by drop-coating.
7. After drying under reduced pressure, the sensor was stored under exclusion of light and moisture.

### Example 5

Further, another first mixed layer was prepared on glass as substrate by using the ether-based hydrophilic polyurethane Hydromed^{™}-D4, designated as HD4 thereinafter, the sulfonic acids, 4-iodobenzenesulfonic acid and 4-toluenesulfonic acid and triphenylamine as described below:
1. 2 g HD4 were dissolved in 22 g EtOH and 2.5 g H₂O.
2. 8 µL HD4-solution, 5.0 mg 4-iodobenzenesulfonic acid, 5.0 mg 4-toluenesulfonic acid and 0.8 mg triphenylamine were mixed with 15 µL EtOH and 15 µL ethylene acetate and ultrasonicated for 30 min at 60 °C.
3. 0.5 µL of the resulting mixture were applied to the substrate by drop-coating.
4. After drying under reduced pressure, the sensor was stored under exclusion of light and moisture.

### Example 6

Further, another first mixed layer was prepared on glass as substrate by using the ether-based hydrophilic polyurethane Hydromed^{™}-D7, designated as HD7 thereinafter, the sulfonic acids, 4-iodobenzenesulfonic acid and 1,5-naphthalenedisulfonic acid, triphenylamine as well as the fluorescent dye rhodamine 6G as described below:
1. 2 g HD7 were dissolved in 22 g EtOH and 2.5 g H₂O (solution A).
2. 5.6 mg triphenylamine were dissolved in 75 µL EtOH and 75 µL ethyl acetate (solution B).
3. 6.9 mg 1,5-naphthalenedisulfonic acid were dissolved in 50 µL EtOH and 50 µL ethyl acetate (solution C).
4. 22.3 mg 4-iodobenzenesulfonic acid were dissolved in 50 µL EtOH and 50 µL ethyl acetate (solution D).
5. 3.1 mg rhodamine 6G were dissolved in 2.1 mL MeOH (solution E).
6. A mixture of 3 µL solution A, 3.4 µL solution B, 15 µL solution C, 30 µL solution D, 20 µL solution E were ultrasonicated for 30 min at 60 °C.
7. 0.3 µL of the mixture were applied to the substrate by drop-coating.
8. After drying under reduced pressure, the sensor was stored under exclusion of light and moisture.

Corresponding graphs of the observed fluorescence intensity are shown in **Figs. 2** through **6C** (corresponding to examples 1 through 6).

**Fig. 2** shows the temporal fluorescence responses of the sensor of example 1. Twice 10 µg TATP were detected, exemplified by a decrease in fluorescence. Also 1 µL water shows a small modulation in fluorescence. 1 µg Hydrogen peroxide shows a drastic decrease in fluorescence. **Fig. 3** shows the temporal fluorescence responses of the sensor of example 2. Again twice 10 µg TATP were measured, which induce a strong decrease in fluorescence. 1 µL water also shows a slight decrease, whereas hydrogen peroxide shows a drastic decrease in fluorescence. **Fig. 4** shows the temporal fluorescence responses of the sensor of example 3. The first 10 µg TATP already induce a drastic decrease in fluorescence, whereas the second 10 µg TATP show just a slight decrease. 1 µL water shows a brief modulation, while hydrogen peroxide shows a drastic decrease. **Fig. 5** shows the temporal fluorescence responses of example 4. **Fig. 6** shows the temporal fluorescence responses of example 5. Twice 10 µg TATP cause both a decrease in fluorescence. 1 µL water increases the fluorescence transiently. 1 µg hydrogen peroxide decreases the fluorescence drastically. **Figs. 6A** and **6B** show the temporal fluorescence responses of example 6. **Fig. 6B** shows the fluorescence decrease on detection of 25, 50 and 100 ng TATP. **Fig. 6C** shows the slow, but drastic decrease of fluorescence on detection of 230 and 460 ng of HMTD.
The following examples correspond to the second aspect:

### Example 7 - Preparation of the analyte sensitive layer according to method 1

Solution 1 = 789 mg Hydromed^{™}-D7 (referred to as HD7 hereinafter) + 8781 mg ethanol + 1043 mg water; solution 2 = 567 mg 4-bromobenzenesulfonic acid monohydrate + 8683 mg solution 1 + 12329 mg ethanol + 1374 mg water + 58.5 mg the triarylborane dye according to structure (14') with R⁶¹ = Me; solution 3 = 11807 mg solution 2 + 7.3 mg 3,5-dinitrosalicylic acid; 0.25-0.5 µL of the solution 3 is drop-coated onto the substrate. Herein the ratio m_{carrier material}/m_{acid} was 1.13.

### Example 8 - Preparation of the catalyst layer according to method 2

Solution 1 = 397 mg Hydromed^{™}-D640 (referred to as HD640 hereinafter) + 4396 mg ethanol + 495 mg water; solution 2 = 24 mg 4-bromobenzenesulfonic acid monohydrate + 56 mg solution 1. 0.25-0.5 µL of the solution 2 were applied to the substrate by drop-coating.

### Example 9a - Preparation of the catalyst layer according to method 3

Solution 1 = 2690 mg 4-bromobenzenesulfonic acid monohydrate + 1459 mg tetrabutylammonium hydroxide solution (40%) in water; suspension 1 = 206 mg fumed silica (0.2 - 0.3 µm) + 504 mg solution 1 + 3249 mg ethanol + 4117 mg water. 2-3 µL of the homogenized suspension were applied to the substrate by drop-coating. In their mixture the components of solution 1 are forming 4-bromobenzenesulfonic acid.

### Example 9b - Preparation of the catalyst layer according to method 4

2-3 µL of the perfluorinated resin Nafion (5% in lower aliphatic alcohols and water, contains 15-20 % water) were applied to the substrate by drop-coating. 2-3 µL of the homogenized suspension/solution were applied to the substrate by drop-coating.

### Example 9 - Detection of TATP with explosive trace:

The peroxide sensor was introduced in an explosive trace detector (prototype) and connected with the analyte/sample admission (heated from 70-160 °C) and with a pump. The peroxides were directed at first to the first mixed layer on position 1 then to the acidic catalyst on position 2 and then to the first mixed layer on position 3. The positions 1 and 3 excited by LEDs at 355-365 nm and the fluorescence intensity changes of the mixed layers were detected.

Quantities of 10 ng-10 µg TATP on wipe sample material teflon were heated up with a hot carrier gas stream (air) to 50-150 °C. The maximal fluorescence intensity is obtained within 6 seconds (cf. **Figs. 10** and **11****,** **13, 14** and **15**). First mixed layers were prepared according to examples 7, 10 and 11 as described above.

The current detection limit for TATP is <10 ng, <100 ng for HMTD, and <8 ng for hydrogen peroxide. When measuring pure water (>1 µL), the analyte sensitive layers show fluorescence quenching. When measuring pure acetone (1 µL), the first mixed layers show no reaction.

### Example 10 - Preparation of first mixed layers comprising HD6 according to above method 1 for detection of peroxides, wherein the ratio m_{carrier material}/m_{acid} = 2.12.

Solution 1 = 204 mg HD6 + 2254 mg ethanol + 254 mg water; solution 2 = 6.6 mg 4-bromobenzenesulfonic acid monohydrate + 186 mg solution 1; solution 3 = 0.1 mg of triarylborane dye according to structure (14') with R⁶¹ = Me + 20 µL solution 2. Finally, 0.25-0.5 µL of the solution 3 has been drop-coated on the substrate.

### Example 11 - Preparation of first mixed layers comprising HD7 according to above method 1, wherein the ratio m_{carrier material}/m_{acid} = 0. 7.

Solution 1 = 194 mg HD7 + 2139 mg ethanol + 245 mg water; solution 2 = 7.6 mg 4-bromobenzenesulfonic acid monohydrate + 72 mg solution 1; solution 3 = 0.1 mg of triarylborane dye according to structure (14') with R⁶¹ = Me + 20 µL solution 2. Finally, 0.25-0.5 µL of the solution 3 has been drop-coated on the substrate.

Thus, according to the first aspect the sensor (fluorescence indicator) for detecting an organic peroxide or its disintegration product comprises a first mixed layer comprising: a molecular probe selected from: triphenylamine, N,N,N',N'-tetraphenylbenzidine, a substance according to formula (11), where R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹, R⁵⁰ and R⁵¹ = H, a substance according to formula (11) with R³², R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷, R⁴⁸, R⁴⁹ and R⁵¹ are identical = H, and the residues R³³ and R⁵⁰ are identical = Me / i.e. N,N'-bis(3-methylphenyl)-N,N'-diphenylbenzidine; a sulfonic acid, selected from: 4-iodobenzenesulfonic acid, 1,5-naphthalenedisulfonic acid, 4-toluenesulfonic acid, and a Nafion^{™}; and a hydrophilic substance selected from: Hydromed^{™}-D4, Hydromed^{™}-D6, Hydromed^{™}-D7, Hydromed^{™}-D640 and/or a tetrabutylammonium, a tetraoctylammonium, or a tetraalkylammonium hydroxide or salt of the indicated sulfonic acid; wherein the first mixed layer is deposited on a substrate, selected from a glass and a COC comprising TOPAS. Instead of the non-iodinated sulfonic acid another fluorescent dye which absorbs at the emission wavelength of the reaction product formed and thus attenuates its fluorescence may be used in order to enhance detection sensitivity.

According to the second aspect the sensor (fluorescence indicator) for detecting an organic peroxide or its disintegration product comprises at least a first mixed layer containing a molecular probe selected from: a triarylborane dye according to formula (14') with R⁶¹ = Me; a sulfonic acid, selected from: 4-iodobenzenesulfonic acid and 4-bromobenzenesulfonic acid monohydrate, 1,5-naphthalenedisulfonic acid, 4-toluenesulfonic acid, and a Nafion^{™}; a hydrophilic substance selected from: Hydromed^{™}-D4, Hydromed^{™}-D6, Hydromed^{™}-D7, Hydromed^{™}-D640 and/or a tetrabutylammonium, a tetraoctylammonium, or a tetraalkylammonium hydroxide or salt of the indicated sulfonic acid and, optionally, 3,5-dinitrosalicylic acid. Further, the sensor according to the second aspect may comprise a separate layer which acts as a catalytic layer and comprises at least a sulfonic acid, selected from: a Nafion^{™} and 4-bromobenzenesulfonic acid monohydrate either alone or with one of the hydrophilic substances indicated or together with a tetrabutylammonium, a tetraoctylammonium, or a tetraalkylammonium hydroxide or a a tetrabutylammonium, a tetraoctylammonium, or a tetraalkylammonium salt of the indicated sulfonic acid. If the sulfonic acid is used together with the hydroxide, a thickener such as fumed silica may be added. According to preferred embodiments, the sensor comprises a first mixed layer as indicated, a catalytic layer and yet another first mixed layer which are arranged sequentially after each other such as to allow when used a stream of hot air carrying the analyte(s) to establish fluidic contact with these layers. The layers are arranged on either glass or on a COC, e.g. TOPAS.

In typical embodiments according to both, first and second aspects, light sources and optical detectors are suitably arranged to detect at the first mixed layers a fluorescent light. In an epi-fluorescence set-up backscattered fluorescent light is detected or, optionally by using a suitable edge filter, the fluorescent light is acquired in a trans-illumination mode.

The synthesis of the molecular probe according to structure (14*) starts from 4-iodophenylboronic acid and 4-{di(mesityl)boronyl}phenylacetylene (acquired as described in J. Phys. Chem. A 2009, 113, 5585-5593) according to the following scheme.

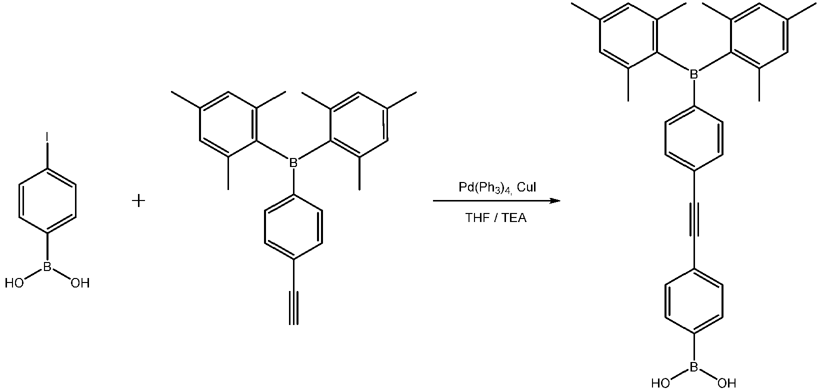

In particular, 4-iodophenylboronic acid (46.5 mg, 0.188 mmol) and 4-{di(mesityl)boronyl}phenylacetylene (70 mg, 0.200 mmol) were solved in dry tetrahydrofuran (THF) (2 mL) and dry triethylamine (TEA) (1 mL). After adding tetrakis(triphenylphosphine)palladium(0) (Pd(Ph₃)₄) (17.2 mg, 0.015 mmol) and copper iodide (CuI) (15.8 mg, 0.083 mmol) the mixture was vigorously stirred at room temperature for 18 hours under a argon atmosphere. When the reaction was completed, the mixture was poured on water (50mL) containing concentrated hydrochloric acid (2mL). The crude product was extracted with dichloromethane (3x 50mL), dried over sodium sulfate, concentrated under reduced pressure and purified by flash silica gel column chromatography (dichloromethane/methanol 5:0.1) to obtain the corresponding product as pale green solid (72.1 mg, yield: 81%). ¹H NMR (600 MHz, CDCl₃, ppm): δ 8.24 (d, *J =* 12Hz, 2H), 7.73 (d, *J =* 12.6Hz, 2H), 7.56 (m, 4H), 6.86 and 6.87 (s, 4H), 2.34 and 2.35 (s, 6H), 2.04 and 2.05 (s, 12H). ¹³C NMR (150 MHz, CDCl₃, ppm): δ 146.3, 141.5, 140.8, 138.9, 136.1, 135.5, 133.2, 131.2, 131.1, 128.3, 127.4, 126.2, 91.9, 91.4, 23.4, 21.2. ¹¹B NMR (192 MHz, CDCl₃, ppm): δ 27.9, 74.5.

Some aspects of the embodiments described herein can briefly be summarized as follows.
1. simple preparation of the sensors respective of the analyte sensitive layers;
2. reaction of peroxides with specially selected molecular probes;
3. detection of peroxides by fluorescence amplification;
4. selective detection of relevant peroxides;
5. quantitative detection of these peroxides;
6. sensitive detection of relevant peroxides;
7. selective detection of important peroxides in a complex mixture;
8. detection of these peroxides in air, in solution and as wipe samples, i.e. from solid surfaces;
9. multiple measurement / simultaneous detection;
10. internal referencing.

The present invention has been explained with reference to various illustrative embodiments and examples. As is apparent to one skilled in the art, the embodiments described herein can be implemented in various ways without departing from the scope of what is invented. Various features, aspects, and functions described in the embodiments can be combined with other embodiments.

## Claims

1. A sensor comprising a substrate and a first mixed layer arranged on the substrate, the first mixed layer comprising:
- a molecular probe;
- a sulfonic acid; and
- a hydrophilic compound;
wherein the molecular probe is selected from a triarylborane.

2. The sensor according to claim 1, wherein the hydrophilic compound comprises a hydrophilic polymer selected from:
- a block copolymer comprising at least a hydrophilic block;
- a hydrophilic polyurethane selected among: an ether-based hydrophilic polyurethane, a hydrophilic thermoplastic polyurethane elastomer and an aliphatic polyether-based thermoplastic polyurethane; and
- an aliphatic polyether-based polyurethane.

3. The sensor according to claims 1-2, wherein the substrate is selected from:
a glass, a metal, a polymer, a mineral, a ceramic, or a composite comprising at least one of them; wherein the polymer comprises a cyclic olefin polymer (COP), a cyclic olefin copolymer (COC), a polyethylene (PE), a polypropylene (PP), a polyvinyl chloride (PVC), a polystyrene (PS), a polytetrafluoroethylene (PTFE),
a polymethylmethacrylate (PMMA), a polyacrylonitrile (PAN), a polyamide, an aramide i.e. aromatic polyamide, a polyetherketone (PEK), a polycarbonate (PC), a polyethylene terephthalate (PET), preferably a cyclic olefin polymer, a cyclic olefin copolymer and/or a polycarbonate.

4. The sensor according to any of claims 1-3, further comprising an excitation light and an optical detector, wherein main optical axes of the excitation light and of the optical detector are arranged with respect to each other within an angle of less than 45 °.

5. The sensor according to claims 1-4, wherein the triarylborane is a substance according to structure (14): 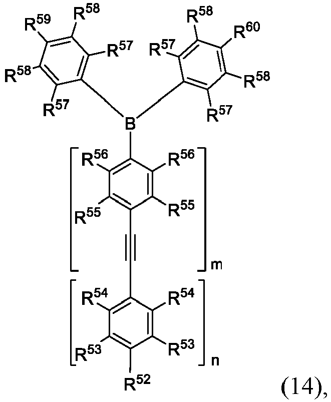 wherein
a bond between the residue R⁵² and the adjacent phenyl group is selected from: a single sigma bond as shown, a phenylene group, a double bond, and a triple bond; and
R⁵² = B(O)₂²⁻, B(OH)(O)⁻, B(OH)₂, B(OH)₃⁻, B(OMe)₂, B(OEt)₂, B(OPr)₂, B(Oalkyl)₂, B(OPh)₂, B(Oaryl)₂ or B(OCH₂CH₂)ₓOR²)₂ wherein R² = H, Me or alkyl with x = 1, 2, 3;
n, m = 0, 1, 2, 3, 4; wherein n+m ≥ 1;
R⁵³, R⁵⁶, R⁵⁷ = H, Me, alkyl, F, Cl, Br, I, CH₂OH, CH₂O(alkyl), CH₂NMe₂, CH₂N(alkyl)₂, CH₂P(t-Bu)₂, CH₂P(alkyl)₂, OMe, OiPr, O(alkyl), O(poly)ethylene oxide;
R⁵⁴, R⁵⁵, R⁵⁸ = H, Me, alkyl, Ph, aryl,F, Cl, Br, I, NMe₂, N(alkyl)₂; and
R⁵⁹, R⁶⁰ = H, Me, alkyl, Ph, aryl, F, Cl, Br, I, CF₃, NMe₂, N(alkyl)₂, 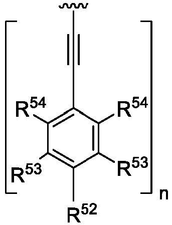 wherein n = 1, 2, 3, 4;
or
R⁵² = a boronic ester, respectively a cyclic boron compound, of glycol, pinacol, 1,4-butanediol, 1,5-pentanediol, tartaric acid, 2,2-dimethyl-1,3-propanediol, a cyclic boronate of methyliminodiacetic acid (MIDA), a cyclic boronamide of brenzcatechine, 1,8-diaminonaphthalene, o-phenylenediamine, or a derivative of them;
wherein
n, m = 0, 1, 2, 3, 4, wherein (n+m) ≥ 1;
R⁵³, R⁵⁶, R⁵⁷ = H, Me, alkyl, F, Cl, Br, I, CH₂OH, CH₂O(alkyl), CH₂NMe₂, CH₂N(alkyl)₂, CH₂P(t-Bu)₂, CH₂P(alkyl)₂, OMe, OiPr, O(alkyl), O(poly)ethylene oxide;
R⁵⁴, R⁵⁵, R⁵⁸ = H, Me, alkyl, Ph, aryl, F, Cl, Br, I, NMe₂, N(alkyl)₂; and
R⁵⁹, R⁶⁰ = H, I, Me, NMe₂, N(alkyl)₂, NPh₂, N(aryl)₂, ethynyl, trimethylsilylethynyl, 1-propynyl, alkynyl, phenylethynyl;
wherein a triple in substance according to structure (14) and in one of the optional residues R⁵⁹ and R⁶⁰ is optionally replaced by one of: a double bond, a phenylene group or a simple sigma bond.

6. The sensor according to claim 1-5, wherein the triarylborane is selected among a substance according to structure (14'), (14") and (14‴): 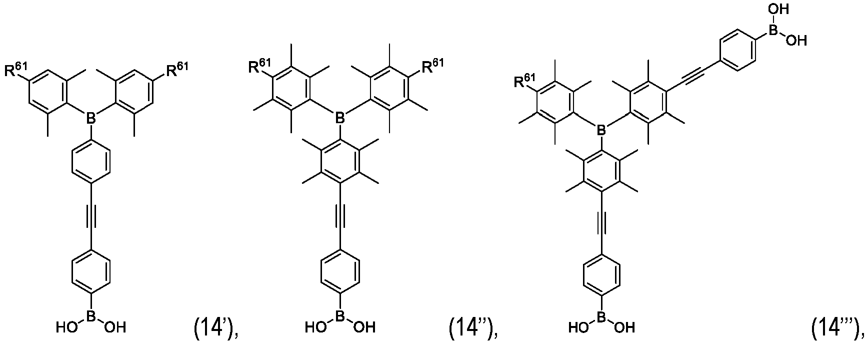 with
R⁶¹ = H, F, Cl, Br, I, Me, alkyl, aryl, vinyl, ethynyl, alkynyl, CF₃, NMe₂, NPh₂, BMes₂.

7. The sensor according to any of claims 1-6 wherein the triarylborane is selected from a substance according to structure (14*): 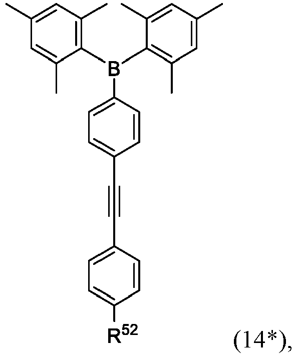 wherein
R⁵² = B(O)₂²⁻, B(OH)(O)⁻, B(OH)₂, B(OH)₃⁻, B(OMe)₂, B(OEt)₂, B(OPr)₂, B(Oalkyl)₂, B(OPh)₂, B(Oaryl)₂, or B(OCH₂CH₂)ₙOR²)₂, wherein R² = H, Me, or alkyl with x = 1, 2, 3;
or
R⁵² = boronic ester respectively cyclic boron compounds of glycol, pinacol, 1,4-butanediol, 1,5-pentanediol, tartaric acid, 2,2-dimethyl-1,3-propanediol, a cyclic boronate of methyliminodiacetic acid (MIDA) i.e. at least one of the cyclic boronates of MIDA, a cyclic boronamide of brenzcatechine, 1,8-diaminonaphthalene, o-phenylenediamine, or derivatives thereof.

8. The sensor according to any of claims 1-7, wherein the triarylborane is a substance according to structure (15) as indicated below:

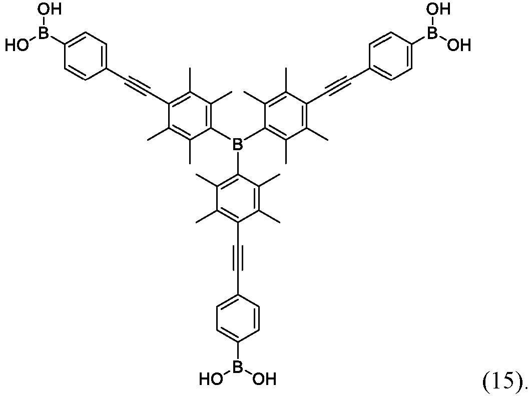

9. The sensor according to any of claims 1-8, wherein the sulfonic acid is selected from a substance according to structure (1), (2) and (3): 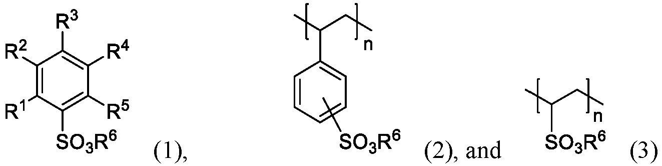
or their mixture, wherein
R¹, R⁵= H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO₃⁻;
R², R⁴= H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO₃⁻;
R³ = H, Me, Et, Pr, alkyl, vinyl, Ph, aryl, F, Cl, Br, I, CF₃, CH₂OH, CO₂H, PO₃H₂, O(alkyl), OPh, O(aryl), OH, OMe, CN, NO₂, NH₂, SO₃H, SO₃⁻, (poly)ethylene oxide,
wherein
n ≥ 8, particularly, 8 ≤ n ≤ 500, preferably: 150 ≤ n ≤ 400;
or
wherein the sulfonic acid is selected from a perfluorosulfonic acid according to structure (6) and (7), or their mixture: 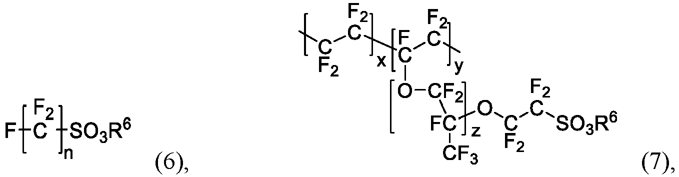 with
R⁶ = H, Na, K;
n ≥ 8,
x = y = 50-150, especially 75-125, and
z= 0, 1, 2, 3;
wherein substances according to structure (7) are known as Nafion^{™}, Hyflon^{™}, Aquivion^{™}, and/or 3M^{™}-ionomer.

10. The sensor according to any of claims 1-9, wherein a second separate layer is arranged adjacent to the first mixed layer and downstream with respect to a fluid stream, the fluid stream potentially containing the analyte, wherein the second separate layer and the first mixed layer are arranged on the same substrate, and wherein a second mixed layer is arranged downstream of the first mixed layer with respect to an applicable fluid stream which carries the analyte; and
wherein the second separate layer comprises:
- a sulfonic acid, or
- a sulfonic acid and a salt of the sulfonic acid, or
- a sulfonic acid and the hydrophilic compound, or
- a sulfonic acid, a salt of the sulfonic acid and the hydrophilic compound;
wherein a cation of the salt of the sulfonic acid is selected from: a sodium, a potassium, an ammonium, a methylammonium, a dimethylammonium, a trimethylammonium, a tetramethylammonium, a tetraethylammonium, a tetrapropylammonium, tetrabutylammonium and/or tetraoctylammonium cation; and wherein the second separate layer does not overlap with the first mixed layer.

11. A method of fabricating a sensor according to any of claims 1-10, comprising:
- mixing a molecular probe, a sulfonic acid, and a hydrophilic compound; and
- forming the first mixed layer of the sensor comprising the mixed molecular probe, the sulfonic acid, and the hydrophilic compound on a substrate;
wherein the molecular probe is selected from:
a triarylborane, and wherein the hydrophilic compound and/or a mixing ratio of the molecular probe, the sulfonic acid, and the hydrophilic compound is/are selected such as to form, optionally upon drying, a free standing film.

12. The method according to claim 11, wherein the molecular probe is selected from a triarylborane according to any of claims 5-8, the method further comprising:
- forming a second separate layer adjacent to the first mixed layer, wherein the second separate layer comprises:
- a sulfonic acid, or
- a sulfonic acid and a salt of the sulfonic acid, or
- a sulfonic acid and the hydrophilic compound, or
- a sulfonic acid, a salt of the sulfonic acid and a hydrophilic compound;
wherein the salt of the sulfonic acid comprises a cation selected from: a sodium, a potassium, an ammonium, a methylammonium, a dimethylammonium, a trimethylammonium, a tetramethylammonium, a tetraethylammonium, a tetrapropylammonium, tetrabutylammonium and/or a tetraoctylammonium cation; and wherein the second separate layer does not overlap with the first mixed layer.

13. The method according to claim 12, wherein the second separate layer further comprises a fluorescence attenuating compound selected from: 3,5-dinitrosalicylic acid, 3,5-dinitrobenzoic acid, 2,4-dinitrosalicylic acid, 2,4-dinitrobenzoic acid, 2,4,6-trinitrobenzoic acid, 2,4-dinitro-6-amino-benzoic acid and their esters, in particular from methylester, ethylester, phenylester and their amides, in particular from N-methylamide, N-ethylamide, N,N-dimethylamide, N,N-diethylamide, N-phenylamide, N,N-diphenylamide.

14. A method of detecting an analyte selected from: triacetone triperoxide, diacetone diperoxide, hexamethylene triperoxide diamine, and hydrogen peroxide;
the method comprising:
- providing at least one sensor according to any of claims 1-10;
- directing a fluid stream comprising the vaporized or gaseous analyte on at least one layer of the sensor, wherein the fluid is preferably air or an inert gas of a temperature within a range
from 15-200 °C, preferably from 50-170 °C;
- exposing the at least one layer to an excitation light, thus exciting a fluorescent compound which is formed within or at the at least one layer upon interaction with the analyte;
- detecting an intensity of a fluorescence; and
- detecting at least one of the analytes qualitatively and/or quantitatively by measuring an enhancement or a decline of a ratio of the intensity.

15. The method according to claim 14, wherein detecting the intensity comprises measuring an attenuation by absorption of at least a part of an excitation light and/or by absorption of at least a part of an emission light, i.e. a fluorescence within or by at least one layer of the sensor.

16. The method according to any of claims 13-14, wherein the sensor further comprises a second separate layer as described in any of claims 12-15 which is arranged downstream of the first mixed layer, wherein triacetone triperoxide is detected by a fluorescence attenuation at the first mixed layer and/or a fluorescence enhancement at the second separate layer.

## Patentansprüche

1. Sensor, der ein Substrat und eine erste Mischschicht aufweist, die auf dem Substrat angeordnet ist, wobei die erste Mischschicht aufweist:
- eine molekulare Sonde;
- eine Sulfonsäure; und
- eine hydrophile Verbindung;
wobei die molekulare Sonde aus einem Triarylboran ausgewählt ist.

2. Sensor nach Anspruch 1, wobei die hydrophile Verbindung ein hydrophiles Polymer aufweist, das ausgewählt ist aus:
- einem Blockcopolymer, das mindestens einen hydrophilen Block aufweist;
- einem hydrophilen Polyurethan, ausgewählt aus: einem hydrophilen Polymer auf Etherbasis, einem hydrophilen Polyurethan auf Etherbasis, einem hydrophilen thermoplastischen Polyurethan-Elastomer und einem aliphatischen thermoplastischen Polyurethan auf Polyetherbasis; und
- einem aliphatischen Polyurethan auf Polyetherbasis.

3. Sensor nach Anspruch 1-2, wobei das Substrat ausgewählt ist aus:
einem Glas, einem Metall, einem Polymer, einem Mineral, einer Keramik oder einem Verbundwerkstoff, der mindestens eines davon aufweist; wobei das Polymer ein cyclisches Olefinpolymer (COP), ein cyclisches Olefincopolymer (COC), ein Polyethylen (PE), ein Polypropylen (PP), ein Polyvinylchlorid (PVC), ein Polystyrol (PS), ein Polytetrafluorethylen (PTFE), ein Polymethylmethacrylat (PMMA), ein Polyacrylnitril (PAN), ein Polyamid, ein Aramid d. h. ein aromatisches Polyamid, ein Polyetherketon (PEK), ein Polycarbonat (PC), ein Polyethylenterephthalat (PET), vorzugsweise ein zyklisches Olefinpolymer, ein zyklisches Olefincopolymer und/oder ein Polycarbonat umfasst.

4. Sensor nach einem der Ansprüche 1 bis 3, der ferner ein Anregungslicht und einen optischen Detektor aufweist, wobei optische Hauptachsen des Anregungslichts und des optischen Detektors in einem Winkel von weniger als 45° zueinander angeordnet sind.

5. Sensor nach einem der Ansprüche 1-4, wobei das Triarylboran eine Substanz der Struktur (14) ist: 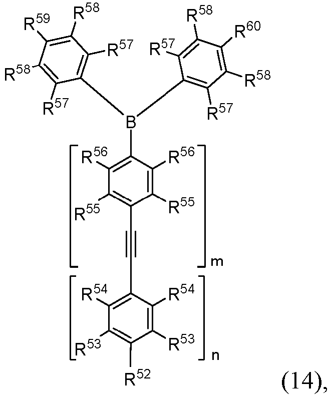 wobei
eine Bindung zwischen dem Rest R⁵² und der benachbarten Phenylgruppe ausgewählt ist aus: einer einfachen Sigma-Bindung wie gezeigt, einer Phenylengruppe, einer Doppelbindung und einer Dreifachbindung; und
R⁵² = B(O)₂²⁻ , B(OH)(O)⁻ , B(OH)₂ , B(OH)₃⁻ , B(OMe)₂ , B(OEt)₂, B(OPr)₂ , B(Oalkyl)₂ , B(OPh)₂ , B(Oaryl)₂
oder B(OCH₂ CH )₂ₓ OR )²₂ wobei R² = H, Me oder Alkyl mit x = 1, 2, 3;
n, m = 0, 1, 2, 3, 4; wobei n+m ≥ 1;
R⁵³ , R⁵⁶ , R⁵⁷ = H, Me, Alkyl, F, Cl, Br, I, CH₂ OH, CH₂ O(Alkyl), CH₂NMe₂, CH₂N(Alkyl)₂ , CH₂P(t-Bu)₂ , CH₂P(Alkyl)₂ , OMe, OiPr, O(Alkyl), O(Poly)ethylenoxid;
R⁵⁴, R⁵⁵, R⁵⁸ = H , Me, Alkyl, Ph, Aryl,F, Cl, Br, I, NMe₂ , N(Alkyl)₂ ; und
R⁵⁹, R⁶⁰ = H, Me, Alkyl, Ph, Aryl, F, Cl, Br, I, CF₃, NMe₂, N(Alkyl)₂, 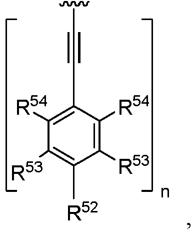 wobei n = 1, 2, 3, 4;
oder
R⁵² = ein Boronsäureester bzw. eine cyclische Borverbindung von Glykol, Pinacol, 1,4-Butandiol, 1,5-Pentandiol, Weinsäure, 2,2-Dimethyl-1,3-propandiol, ein zyklisches Boronat der Methyliminodiessigsäure (MIDA), ein zyklisches Boronamid des Brenzcatechins, 1,8-Diaminonaphthalin, o-Phenylendiamin oder ein Derivat davon;
wobei
n, m = 0, 1, 2, 3, 4, wobei (n+m) ≥ 1;
R⁵³ , R⁵⁶ , R⁵⁷ = H, Me, Alkyl, F, Cl, Br, I, CH₂ OH, CH₂ O(Alkyl), CH NMe₂₂, CH₂ N(Alkyl)₂ , CH₂P(t-Bu)₂ , CH₂P(Alkyl)₂ , OMe, OiPr, O(Alkyl), O(Poly)ethylenoxid;
R⁵⁴, R⁵⁵, R⁵⁸ = H, Me, Alkyl, Ph, Aryl, F, Cl, Br, I, NMe₂ , N(Alkyl)₂ ; und
R⁵⁹, R⁶⁰= H, I, Me, NMe₂ , N(Alkyl)₂ , NPH₂ , N(Aryl)₂ , Ethynyl, Trimethylsilylethynyl, 1-Propynyl, Alkynyl, Phenylethynyl;
wobei ein Tripel in der Substanz gemäß der Struktur (14) und in einem der fakultativen Reste R⁵⁹ und R⁶⁰ gegebenenfalls durch eine Doppelbindung, eine Phenylengruppe oder eine einfache Sigma-Bindung ersetzt ist.

6. Sensor nach Anspruch 1-5, wobei das Triarylboran ausgewählt ist aus einer Substanz gemäß Struktur (14'), (14") und (14‴): 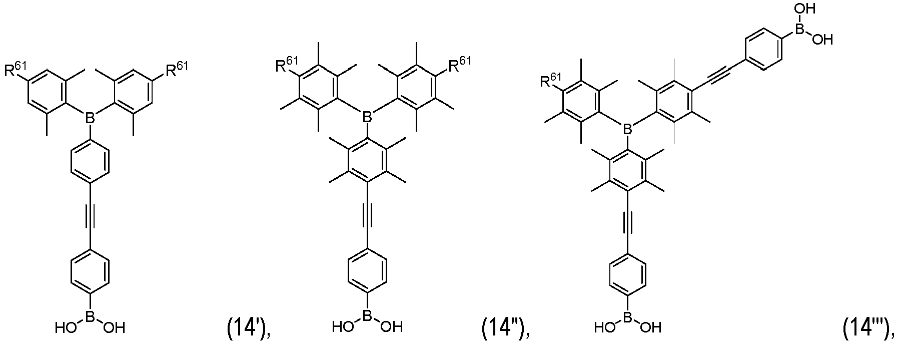 mit
R⁶¹ = H, F, Cl, Br, I, Me, Alkyl, Aryl, Vinyl, Ethinyl, Alkinyl, CF₃, NMe₂ , NPH₂ , BMes₂ .

7. Sensor nach einem der Ansprüche 1-6, wobei das Triarylboran aus einer Substanz der Struktur (14*) ausgewählt ist: 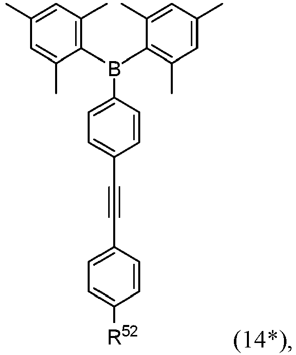 wobei
R⁵² = B(O)₂²⁻, B(OH)(O)-, B(OH)₂ , B(OH)₃⁻, B(OMe)₂, B(OEt)₂, B(OPr)₂, B(Oalkyl)₂, B(OPh)₂, B(Oaryl)₂, oder B(OCH₂ CH )₂ₙ OR )²₂ , wobei R² = H, Me, oder Alkyl mit x = 1, 2, 3;
oder
R⁵² = Boronsäureester bzw. zyklische Borverbindungen von Glykol, Pinacol, 1,4-Butandiol, 1,5-Pentandiol, Weinsäure, 2,2-Dimethyl-1,3-propandiol, ein zyklisches Boronat von Methyliminodiessigsäure (MIDA), d.h. mindestens eines der zyklischen Boronate von MIDA, ein zyklisches Boronamid von Brenzcatechin, 1,8-Diaminonaphthalin, o-Phenylendiamin oder Derivate davon.

8. Sensor nach einem der Ansprüche 1 bis 7, wobei das Triarylboran eine Substanz gemäß der Struktur (15) ist, wie unten angegeben:

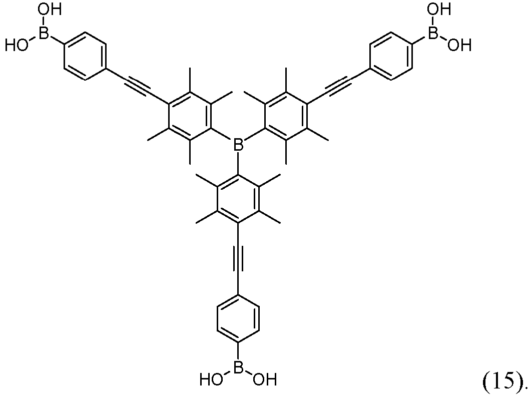

9. Sensor nach einem der Ansprüche 1 bis 8, wobei die Sulfonsäure aus einer Substanz gemäß Struktur (1), (2) und (3) ausgewählt ist: 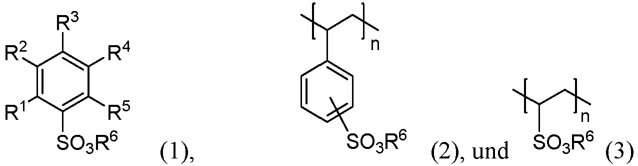
oder deren Gemisch, wobei
R¹, R⁵ = H, Me, Et, Pr, Alkyl, Vinyl, Ph, Aryl, F, Cl, Br, I, CF₃, CH₂OH, CO₂H, PO₃H₂, O(Alkyl), OPh, O(Aryl), OH, OMe, CN, NO₂, NH₂, SO₃H, SO₃⁻,
R², R⁴ = H, Me, Et, Pr, Alkyl, Vinyl, Ph, Aryl, F, Cl, Br, I, CF₃, CH₂OH, CO₂ H, PO₃H₂, O(Alkyl), OPh, O(Aryl), OH, OMe, CN, NO₂, NH₂, SO₃H, SO₃⁻,
R³ = H, Me, Et, Pr, Alkyl, Vinyl, Ph, Aryl, F, Cl, Br, I, CF₃, CH₂OH, CO₂H, PO₃H₂, O(Alkyl), OPh, O(Aryl), OH, OMe, CN, NO₂, NH₂, SO₃H, SO₃ , (Poly)ethylenoxid,
wobei
n ≥ 8, insbesondere, 8 ≤ n ≤ 500, vorzugsweise: 150 ≤ n ≤ 400;
oder
wobei die Sulfonsäure ausgewählt ist aus einer Perfluorsulfonsäure gemäß Struktur (6) und (7) oder deren Gemisch: 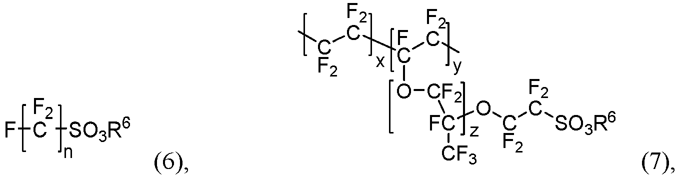 mit
R⁶ = H, Na, K;
n ≥ 8,
x = y = 50-150, insbesondere 75-125, und
z = 0, 1, 2, 3,
wobei Stoffe gemäß Struktur (7) als Nafion^{™}, Hyflon^{™}, Aquivion^{™}, und/oder 3M^{™}-Ionomer bekannt sind.

10. Sensor nach einem der Ansprüche 1 bis 9, wobei eine zweite separate Schicht neben der ersten gemischten Schicht und stromabwärts in Bezug auf einen Fluidstrom angeordnet ist, wobei der Fluidstrom möglicherweise den Analyten enthält, wobei die zweite separate Schicht und die erste gemischte Schicht auf demselben Substrat angeordnet sind und wobei eine zweite gemischte Schicht stromabwärts der ersten gemischten Schicht in Bezug auf einen anwendbaren Fluidstrom angeordnet ist, der den Analyten trägt; und
wobei die zweite separate Schicht Folgendes aufweist:
- eine Sulfonsäure, oder
- eine Sulfonsäure und ein Salz der Sulfonsäure, oder
- eine Sulfonsäure und die hydrophile Verbindung, oder
- eine Sulfonsäure, ein Salz der Sulfonsäure und die hydrophile Verbindung;
wobei ein Kation des Sulfonsäuresalzes ausgewählt ist aus: einem Natrium, einem Kalium, einem Ammonium, einem Methylammonium, einem Dimethylammonium, einem Trimethylammonium, einem Tetramethylammonium, einem Tetraethylammonium, ein Tetrapropylammonium-, Tetrabutylammonium- und/oder Tetraoctylammonium-Kation;
wobei sich die zweite separate Schicht nicht mit der ersten gemischten Schicht überlappt.

11. Verfahren zur Herstellung eines Sensors nach einem der Ansprüche 1-10, das Folgendes aufweist:
- Mischen einer molekularen Sonde, einer Sulfonsäure und einer hydrophilen Verbindung; und
- Bildung der ersten gemischten Schicht des Sensors, die die gemischte molekulare Sonde, die Sulfonsäure und die hydrophile Verbindung aufweist, auf einem Substrat;
wobei die molekulare Sonde ausgewählt ist aus:
einem Triarylboran, und wobei die hydrophile Verbindung und/oder ein Mischungsverhältnis der molekularen Sonde, der Sulfonsäure und der hydrophilen Verbindung so ausgewählt ist/sind, dass sie, gegebenenfalls beim Trocknen, einen freistehenden Film bilden.

12. Verfahren nach Anspruch 11, wobei die molekulare Sonde ausgewählt ist aus einem Triarylboran nach einem der Ansprüche 5 bis 8, wobei das Verfahren ferner aufweist:
- Bilden einer zweiten separaten Schicht angrenzend an die erste gemischte Schicht, wobei die zweite separate Schicht folgendes aufweist:
- eine Sulfonsäure, oder
- eine Sulfonsäure und ein Salz der Sulfonsäure, oder
- eine Sulfonsäure und der hydrophilen Verbindung, oder
- eine Sulfonsäure, ein Salz der Sulfonsäure und eine hydrophile Verbindung;
wobei das Salz der Sulfonsäure ein Kation aufweist, das ausgewählt ist aus: einem Natrium-, einem Kalium-, einem Ammonium-, einem Methylammonium-, einem Dimethylammonium-, einem Trimethylammonium-, einem Tetramethylammonium-, einem Tetraethylammonium-, einem Tetrapropylammonium-, einem Tetrabutylammonium- und/oder einem Tetraoctylammonium-Kation; und wobei die zweite separate Schicht die erste Mischschicht nicht überlappt.

13. Verfahren nach Anspruch 12, wobei die zweite separate Schicht ferner eine fluoreszenzdämpfende Verbindung aufweist, ausgewählt aus: 3,5-Dinitrosalicyl Säure, 3,5-Dinitrobenzoesäure, 2,4-Dinitrosalicylsäure, 2,4-Dinitrabenzoesäure, 2,4,6-Trinitrobenzoesäure, 2,4-Dinitro-6-amino-benzoesäure und deren Ester, insbesondere aus Methylester, Ethylester, Phenylester und deren Amiden, insbesondere von N-Methylamid, N-Ethylamid, N,N-Dimethylamid, N,N-Diethylamid, N-Phenylamid, N,N-Diphenylamid.

14. Verfahren zum Nachweis eines Analyten, ausgewählt aus: Triacetontriperoxid, Diacetondiperoxid, Hexamethylentriperoxiddiamin und Wasserstoffperoxid, wobei das Verfahren aufweist:
- Bereitstellen mindestens eines Sensors nach einem der Ansprüche 1-10;
- Richten eines Fluidstroms, der den verdampften oder gasförmigen Analyten aufweist, auf mindestens eine Schicht des Sensors, wobei das Fluid vorzugsweise Luft oder ein Inertgas mit einer Temperatur in einem Bereich von 15-200 °C, vorzugsweise von 50-170 °C, ist;
- Belichten der mindestens einen Schicht mit einem Anregungslicht, wodurch eine fluoreszierende Verbindung angeregt wird, die sich in oder an der mindestens einen Schicht bei Wechselwirkung mit dem Analyten bildet;
- Erfassen einer Intensität einer Fluoreszenz; und
- Erfassen mindestens eines der Analyten qualitativ und/oder quantitativ durch Messung einer Zunahme oder Abnahme des Intensitätsverhältnisses.

15. Verfahren nach Anspruch 13, wobei das Erfassen der Intensität die Messung einer Abschwächung durch Absorption mindestens eines Teils eines Anregungslichts und/oder durch Absorption mindestens eines Teils eines Emissionslichts, d. h. einer Fluoreszenz, in oder durch mindestens eine Schicht des Sensors aufweist.

16. Verfahren nach einem der Ansprüche 13-14, wobei der Sensor weiterhin eine zweite separate Schicht, wie in einem der Ansprüche 12 bis 14 beschrieben, aufweist, die stromabwärts der ersten gemischten Schicht angeordnet ist, wobei Triacetontriperoxid durch eine Fluoreszenzabschwächung an der ersten gemischten Schicht und/oder eine Fluoreszenzverstärkung an der zweiten separaten Schicht erfasst wird.

## Revendications

1. Capteur comprenant un substrat et une première couche mixte disposée sur le substrat, la première couche mixte comprenant :
- une sonde moléculaire ;
- un acide sulfonique ; et
- un composé hydrophile ;
dans lequel la sonde moléculaire est choisie parmi les triarylboranes.

2. Capteur selon la revendication 1, dans lequel le composé hydrophile comprend un polymère hydrophile choisi parmi :
- un copolymère séquencé comprenant au moins un bloc hydrophile ;
- un polyuréthane hydrophile choisi parmi : un polyuréthane hydrophile à base d'éther, un élastomère de polyuréthane thermoplastique hydrophile, et un polyuréthane thermoplastique à base de polyéther aliphatique ; et
- un polyuréthane à base de polyéther aliphatique.

3. Capteur selon les revendications 1 et 2, dans lequel le substrat est choisi parmi :
un verre, un métal, un polymère, un minéral, une céramique, et un composite comprenant au moins l'un d'entre eux ; parmi lesquels le polymère comprend un polymère d'oléfine cyclique (COP), un copolymère d'oléfine cyclique (COC), un polyéthylène (PE), un polypropylène (PP), un poly(chlorure de vinyle) (PVC), un polystyrène (PS), un polytétrafluoroéthylène (PTFE), un poly(méthacrylate de méthyle) (PMMA), un polyacrylonitrile (PAN), un polyamide, un aramide, c'est-à-dire un polyamide aromatique, une polyéthercétone (PEK), un polycarbonate (PC), un poly(téréphtalate d'éthylène) (PET), de préférence un polymère d'oléfine cyclique, un copolymère d'oléfine cyclique et/ou un polycarbonate.

4. Capteur selon l'une quelconque des revendications 1 à 3, comprenant en outre une lumière d'excitation et un détecteur optique, dans lequel les axes optiques principaux de la lumière d'excitation et du détecteur optique sont disposés l'un par rapport à l'autre selon un angle inférieur à 45°.

5. Capteur selon les revendications 1 à 4, dans lequel le triarylborane est une substance de structure (14) : 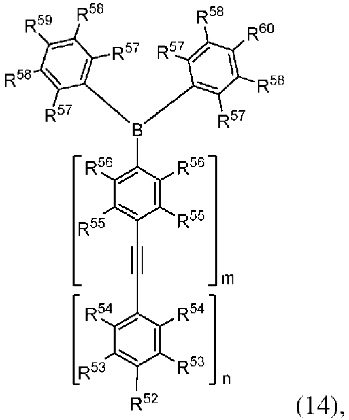 dans laquelle
la liaison entre le résidu R⁵² et le groupe phényle adjacent est choisie parmi :
une liaison simple sigma telle que représentée, un groupe phénylène, une double liaison, et une triple liaison ; et
R⁵² = B(O)₂²⁻, B(OH)(O)⁻, B(OH)₂, B(OH)₃⁻, B(OMe)₂, B(OEt)₂, B(OPr)₂, B(O-alkyle)₂, B(OPh)₂, B(O-aryle)₂ et B(OCH₂CH₂)ₓOR²)₂ où R² = H, Me ou alkyle et x = 1, 2, 3 ;
n, m = 0, 1, 2, 3, 4 ; et n+m ≥ 1 ;
R⁵³, R⁵⁶, R⁵⁷ = H, Me, alkyle, F, Cl, Br, I, CH₂OH, CH₂O(alkyle), CH₂NMe₂, CH₂N(alkyle)₂, CH₂P(t-Bu)₂, CH₂P(alkyle)₂, OMe, OiPr, O(alkyle), O(poly)oxyéthylène ;
R⁵⁴, R⁵⁵, R⁵⁸ = H, Me, alkyle, Ph, aryle, F, Cl, Br, I, NMe₂, N(alkyle)₂ ; et
R⁵⁹, R⁶⁰ = H, Me, alkyle, Ph, aryle, F, Cl, Br, I, CF₃, NMe₂, N(alkyle)₂, 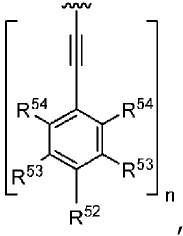 où n = 1, 2, 3, 4 ;
ou bien
R⁵² = un ester boronate, respectivement un composé cyclique du bore et de glycol, pinacol, 1,4-butanediol, 1,5-pentanediol, acide tartrique, 2,2-diméthyl-1,3-propanediol, un boronate cyclique d'acide méthyliminodiacétique (MIDA), un boronamide cyclique de brenzcatéchine, 1,8-diaminonaphtalène, o-phénylènediamine, ou un de leurs dérivés ;
dans laquelle
n, m = 0, 1, 2, 3, 4, où (n+m) ≥ 1 ;
R⁵³, R⁵⁶, R⁵⁷ = H, Me, alkyle, F, Cl, Br, I, CH₂OH, CH₂O(alkyle), CH₂NMe₂, CH₂N(alkyle)₂, CH₂P(t-Bu)₂, CH₂P(alkyle)₂, OMe, OiPr, O(alkyle), O(poly)oxyéthylène ;
R⁵⁴, R⁵⁵, R⁵⁸ = H, Me, alkyle, Ph, aryle, F, Cl, Br, I, NMe₂, N(alkyle)₂ ; et
R⁵⁹, R⁶⁰ = H, I, Me, NMe₂, N(alkyle)₂, NPh₂, N(aryle)₂, éthynyle, triméthylsilyléthynyle, 1-propynyle, alcynyle, phényléthynyle ;
dans lequel une triple liaison dans la substance de structure (14) et dans un des résidus R⁵⁹ et R⁶⁰ optionnels est éventuellement remplacée par : une double liaison, un groupe phénylène ou une liaison simple sigma.

6. Capteur selon les revendications 1 à 5, dans lequel le triarylborane est choisi parmi les substances de structures (14'), (14") et (14‴) : 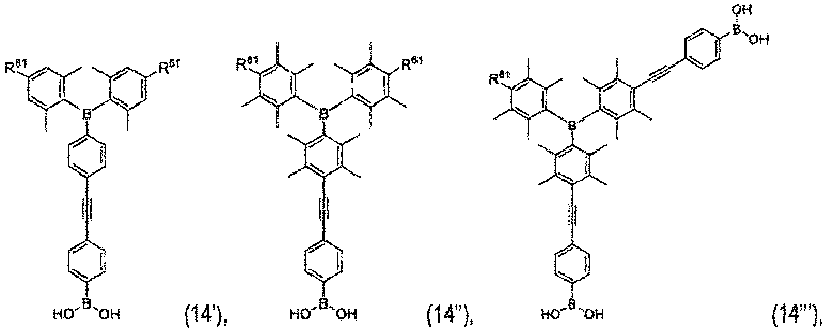 dans lesquelles
R⁶¹ = H, F, Cl, Br, I, Me, alkyle, aryle, vinyle, éthynyle, alcynyle, CF₃, NMe₂, NPh₂, BMes₂.

7. Capteur selon l'une quelconque des revendications 1 à 6, dans lequel le triarylborane est choisi parmi les substances de structure (14*) : 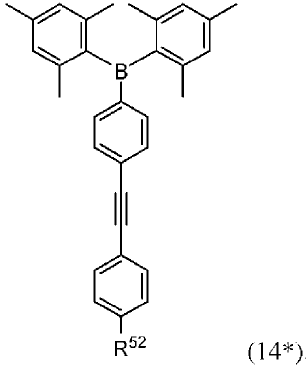 dans laquelle
R⁵² = B(O)₂²⁻, B(OH)(O)⁻, B(OH)₂, B(OH)₃⁻, B(OMe)₂, B(OEt)₂, B(OPr)₂, B(O-alkyle)₂, B(OPh)₂, B(O-aryle)₂, et B(OCH₂CH₂)ₙOR²)₂ où R² = H, Me ou alkyle et x = 1, 2, 3 ;
ou bien
R⁵² = un ester boronate, respectivement un composé cyclique du bore et de glycol, pinacol, 1,4-butanediol, 1,5-pentanediol, acide tartrique, 2,2-diméthyl-1,3-propanediol, un boronate cyclique d'acide méthyliminodiacétique (MIDA), c'est-à-dire au moins l'un des boronates cycliques de MIDA, un boronamide cyclique de brenzcatéchine, 1,8-diaminonaphtalène, o-phénylènediiamine, ou un de leurs dérivés.

8. Capteur selon l'une quelconque des revendications 1 à 7, dans lequel le triarylborane est une substance de structure (15) telle qu'indiquée ci-dessous :

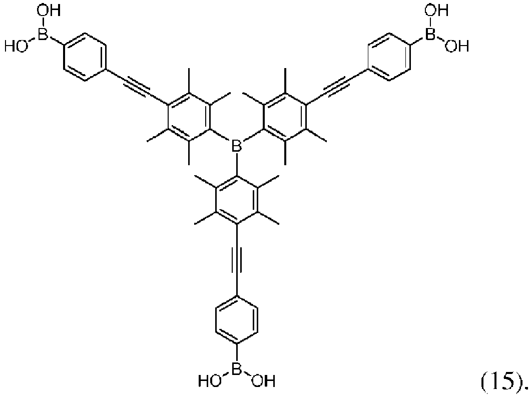

9. Capteur selon l'une quelconque des revendications 1 à 8, dans lequel l'acide sulfonique est choisi parmi les substances de structures (1), (2) et (3) : 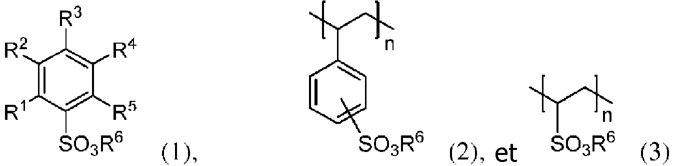 et leurs mélanges, dans lesquelles
R¹, R⁵ = H, Me, Et, Pr, alkyle, vinyle, Ph, aryle, F, Cl, Br, I, CF₃, CH₂OH, CO₂H, PO₃H₂, O(alkyle), OPh, O(aryle), OH, OMe, CN, NO₂, NH₂, SO₃H, SO₃⁻ ;
R², R⁴ = H, Me, Et, Pr, alkyle, vinyle, Ph, aryle, F, Cl, Br, I, CF₃, CH₂OH, CO₂H, PO₃H₂, O(alkyle), OPh, O(aryle), OH, OMe, CN, NO₂, NH₂, SO₃H, SO₃⁻ ;
R³ = H, Me, Et, Pr, alkyle, vinyle, Ph, aryle, F, Cl, Br, I, CF₃, CH₂OH, CO₂H, PO₃H₂, O(alkyle), OPh, O(aryle), OH, OMe, CN, NO₂, NH₂, SO₃H, SO₃⁻, (poly)oxyéthylène,
dans lesquelles
n ≥ 8, en particulier 8 ≤ n ≤ 500, de préférence 150 ≤ n ≤ 400 ;
ou bien
dans lequel l'acide sulfonique est choisi parmi les acides perfluorosulfoniques de structures (6) et (7), et leurs mélanges : 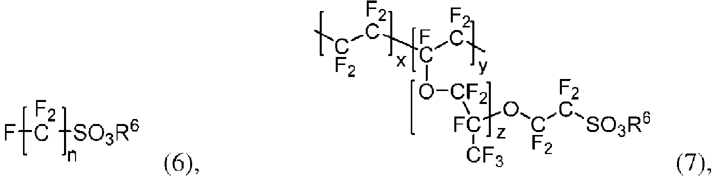 dans lesquelles
R⁶ = H, Na, K ;
n ≥ 8,
x = y = 50 à 150, en particulier 75 à 125, et
z = 0, 1, 2, 3 ;
dans lequel les substances de structure (7) sont connues sous les désignations Nafion^{™}, Hyflon^{™}, Aquivion^{™}, et/ou 3M^{™}-ionomer.

10. Capteur selon l'une quelconque des revendications 1 à 9, dans lequel une deuxième couche séparée est disposée adjacente à la première couche mixte et en aval par rapport à un courant de fluide, le courant de fluide contenant potentiellement l'analyte, dans lequel la deuxième couche séparée et la première couche mixte sont disposées sur le même substrat, et dans lequel une deuxième couche mixte est disposée en aval de la première couche mixte par rapport à un courant de fluide applicable qui transporte l'analyte ; et
dans lequel la deuxième couche séparée comprend :
- un acide sulfonique, ou
- un acide sulfonique et un sel de l'acide sulfonique, ou
- un acide sulfonique et le composé hydrophile, ou
- un acide sulfonique, un sel de l'acide sulfonique et le composé hydrophile ;
dans lequel le cation du sel de l'acide sulfonique est choisi parmi les cations sodium, potassium, ammonium, méthylammonium, diméthylammonium, triméthylammonium, tétraméthylammonium, tétraéthylammonium, tétrapropylammonium, tétrabutylammonium et/ou tétraoctylammonium ; et dans lequel la deuxième couche séparée ne se superpose pas à la première couche mixte.

11. Procédé de fabrication d'un capteur de l'une quelconque des revendications 1 à 10, comprenant :
- le mélange d'une sonde moléculaire, d'un acide sulfonique, et d'un composé hydrophile ; et
- la formation de la première couche mixte du capteur comprenant le mélange de la sonde moléculaire mixte, de l'acide sulfonique, et du composé hydrophile sur un substrat ;
dans lequel la sonde moléculaire est choisie parmi :
les triarylboranes, et dans lequel le composé hydrophile et/ou le rapport de mélange de la sonde moléculaire, de l'acide sulfonique, et du composé hydrophile est/sont choisis de façon à former, éventuellement suite à un séchage, un film autoportant.

12. Procédé selon la revendication 11, dans lequel la sonde moléculaire est choisie parmi les triarylboranes des revendications 5 à 8, le procédé comprenant en outre :
- la formation d'une deuxième couche séparée adjacente à la première couche mixte, laquelle deuxième couche séparée comprend :
- un acide sulfonique, ou
- un acide sulfonique et un sel de l'acide sulfonique, ou
- un acide sulfonique et le composé hydrophile, ou
- un acide sulfonique, un sel de l'acide sulfonique et un composé hydrophile ;
dans lequel le sel de l'acide sulfonique comprend un cation choisi parmi les cations sodium, potassium, ammonium, méthylammonium, diméthylammonium, triméthylammonium, tétraméthylammonium, tétraéthylammonium, tétrapropylammonium, tétrabutylammonium et/ou tétraoctylammonium ; et
dans lequel la deuxième couche séparée ne se superpose pas à la première couche mixte.

13. Procédé selon la revendication 12, dans lequel la deuxième couche séparée comprend en outre un composé atténuateur de fluorescence choisi parmi : l'acide 3,5-dinitrosalicylique, l'acide 3,5-dinitrobenzoïque, l'acide 2,4-dinitrosalicylique, l'acide 2,4-dinitrobenzoïque, l'acide 2,4,6-trinitrobenzoïque, l'acide 2,4-dinitro-6-aminobenzoïque et leurs esters, en particulier l'ester méthylique, l'ester éthylique, l'ester phénylique et leurs amides, en particulier le N-méthylamide, le N-éthylamide, le N,N-diméthylamide, le N,N-diéthylamide, le N-phénylamide, le N,N-diphénylamide.

14. Procédé de détection d'un analyte choisi parmi : le triperoxyde de triacétone, le diperoxyde de diacétone, l'hexaméthylènetriperoxydediamine, et le peroxyde d'hydrogène ; le procédé comprenant :
- la fourniture d'au moins un capteur de l'une quelconque des revendications 1 à 10 ;
- l'amenée d'un courant de fluide comprenant l'analyte vaporisé ou gazeux sur au moins une couche du capteur, lequel fluide est de préférence de l'air ou un gaz inerte à une température située dans la plage allant de 15 à 200°C, de préférence de 50 à 170°C ;
- l'exposition de l'au moins une couche à une lumière d'excitation, ce qui excite ainsi un composé fluorescent qui est formé à l'intérieur ou au niveau de l'au moins une couche suite à une interaction avec l'analyte ;
- la détection d'une intensité d'une fluorescence ; et
- la détection d'au moins un des analytes qualitativement et/ou quantitativement par mesure de l'augmentation ou de la diminution de la proportion d'intensité.

15. Procédé selon la revendication 14, dans lequel la détection de l'intensité comprend la mesure de l'atténuation par absorption d'au moins une partie d'une lumière d'excitation et/ou par absorption d'au moins une partie d'une lumière d'émission, c'est-à-dire d'une fluorescence à l'intérieur ou au niveau d'au moins une couche du capteur.

16. Procédé selon l'une quelconque des revendications 13 et 14, dans lequel le capteur comprend en outre une deuxième couche séparée comme décrit dans l'une quelconque des revendications 12 à 15 qui est disposée en aval de la première couche mixte, dans lequel le triperoxyde de triacétone est détecté par une atténuation de fluorescence au niveau de la première couche mixte et/ou une amplification de fluorescence au niveau de la deuxième couche séparée.
